# EUROPÄISCHE PATENTANMELDUNG

(11) **EP 1 136 504 A1**
(43) Veröffentlichungstag der Anmeldung: **26.09.2001**
(21) Anmeldenummer: 01104578.8
(22) Anmeldetag: 06.03.2001
(51) Int. Cl.: C07K 14/705, G01N 33/566, G01N 33/94, C12N 15/63, C12N 15/85, A01K 67/033, C07K 14/435

(54) **Rezeptor für Latrotoxin aus Insekten**

(30) Priorität: 18.03.2000 DE 10013580
(71) Anmelder: Bayer Aktiengesellschaft, 51368 Leverkusen (DE)
(72) Erfinder: Antonicek, Horst-Peter, Dr., 51467 Bergisch Gladbach (DE); Friedrich, Gabi, Dr., 51377 Leverkusen (DE); Schulte, Thomas, Dr., 51061 Köln (DE)

(57) **Zusammenfassung**

Die Erfindung betrifft Polypeptide, welche die biologische Aktivität von Latrotoxin-Rezeptoren ausüben, sowie Nukleinsäuren, die für diese Polypeptide codieren und insbesondere deren Verwendung zum Auffinden von Wirkstoffen für den Pflanzenschutz.

SEQUENZPROTOKOLL

<110> Bayer Aktiengesellschaft

<120> Rezeptor für Latrotoxin aus Insekten

<130> Le A 34 402

<140>
<141>

<150> DE 100 13 580.3
<151> 2000-03-18

<160> 6

<170> PatentIn Ver. 2.1

<210> 1
<211> 4341
<212> DNA
<213> Drosophila melanogaster

<220>
<221> CDS
<222> (1)..(4341)

<400> 1
atg ata cat aag ttg aat ggt act ttt gag tcc aac ttt cat gaa tat 48
Met Ile His Lys Leu Asn Gly Thr Phe Glu Ser Asn Phe His Glu Tyr
1               5                   10                  15

gac tcc aaa cgg aaa tac ata aga gtg tcc aag tac caa acc gcc tac 96
Asp Ser Lys Arg Lys Tyr Ile Arg Val Ser Lys Tyr Gln Thr Ala Tyr
            20                  25                  30

gcc tgc gaa ggt aag aaa ctg acc atc gag tgc gat ccc ggc gat gtg 144
Ala Cys Glu Gly Lys Lys Leu Thr Ile Glu Cys Asp Pro Gly Asp Val
        35                  40                  45

atc aac ctc att cgg gcc aac tat ggc cgc ttc tcg att acc atc tgc 192
Ile Asn Leu Ile Arg Ala Asn Tyr Gly Arg Phe Ser Ile Thr Ile Cys
    50                  55                  60

aat gac cac ggg aat gtg gag tgg agt gtt aac tgc atg ttt ccc aag 240
Asn Asp His Gly Asn Val Glu Trp Ser Val Asn Cys Met Phe Pro Lys
65                  70                  75                  80

tca ctc agc gta ctg aac tca aga tgt gcc cac aag cag agc tgc ggc 288
Ser Leu Ser Val Leu Asn Ser Arg Cys Ala His Lys Gln Ser Cys Gly
                85                  90                  95

gtg ttg gca gcc acg agc atg ttc ggg gat ccc tgt ccc ggt acc cac 336
Val Leu Ala Ala Thr Ser Met Phe Gly Asp Pro Cys Pro Gly Thr His
            100                 105                 110

aag tat ctg gag gca cac tac cag tgc ata agt gca gcc caa act tcg 384
Lys Tyr Leu Glu Ala His Tyr Gln Cys Ile Ser Ala Ala Gln Thr Ser
        115                 120                 125

acg acg acc aac agg ccc agt ccg ccg cca tgg gtg ctg agc aat ggt 432
Thr Thr Thr Asn Arg Pro Ser Pro Pro Pro Trp Val Leu Ser Asn Gly
    130                 135                 140

ccg ccg atc ttt ggc aac ggc agt gga ctg atc cat ccg ccc ggg gtt 480
Pro Pro Ile Phe Gly Asn Gly Ser Gly Leu Ile His Pro Pro Gly Val
145                 150                 155                 160

gga gcg ggt gcg ccg ccc ccg ccg aga ctt ccc aca ctt ccc gga gtg 528
Gly Ala Gly Ala Pro Pro Pro Pro Arg Leu Pro Thr Leu Pro Gly Val
                165                 170                 175

gtg gga atc agt ggg aat ccc ggc ctg ttc aac gta cca ccg caa cac 576
Val Gly Ile Ser Gly Asn Pro Gly Leu Phe Asn Val Pro Pro Gln His
            180                 185                 190

acc gcc gtc acg cac tcc acg ccc tcg agc agc acg aca gcc gtg ggc 624
Thr Ala Val Thr His Ser Thr Pro Ser Ser Ser Thr Thr Ala Val Gly
        195                 200                 205

ggt gga cgt ttg aag ggt ggg gcc acc tcc acg acg acc acc aag cat 672
Gly Gly Arg Leu Lys Gly Gly Ala Thr Ser Thr Thr Thr Thr Lys His
    210                 215                 220

ccg gct ggc cgc cat gat ggt ctg cca ccg ccg ccg caa ctg cac cac 720
Pro Ala Gly Arg His Asp Gly Leu Pro Pro Pro Pro Gln Leu His His
225                 230                 235                 240

cac cac aac cac cac ggt gaa gac act gcc tca ccc acc aag ccg agc 768
His His Asn His His Gly Glu Asp Thr Ala Ser Pro Thr Lys Pro Ser
                245                 250                 255

agc aag ctg ccg gct ggc ggt aat gcc act tca cca tcc aac acg agg 816
Ser Lys Leu Pro Ala Gly Gly Asn Ala Thr Ser Pro Ser Asn Thr Arg
            260                 265                 270

ata ctc acg ggc gtc gga ggt tcc gga act gat gac gga acc cta ctg 864
Ile Leu Thr Gly Val Gly Gly Ser Gly Thr Asp Asp Gly Thr Leu Leu
        275                 280                 285

acc aca aag agc tca ccc aac cgc cca ccg ggc act gcg gcc agt gga 912
Thr Thr Lys Ser Ser Pro Asn Arg Pro Pro Gly Thr Ala Ala Ser Gly
    290                 295                 300

tcc gtt gtc ccc ggg aac ggc agc gtg gtg cgc acc atc aac aat att 960
Ser Val Val Pro Gly Asn Gly Ser Val Val Arg Thr Ile Asn Asn Ile
305                 310                 315                 320

aat ttg aac gca gcc ggg atg tcc gga ggc gat gat gag tcc aag ttg 1008
Asn Leu Asn Ala Ala Gly Met Ser Gly Gly Asp Asp Glu Ser Lys Leu
                325                 330                 335

ttt tgc ggc ccc act cat gcc cgc aat ttg tac tgg aac atg act cga 1056
Phe Cys Gly Pro Thr His Ala Arg Asn Leu Tyr Trp Asn Met Thr Arg
            340                 345                 350

gtg ggt gat gtg aat gtt cag ccc tgt cct ggc gga gca gcc ggc atc 1104
Val Gly Asp Val Asn Val Gln Pro Cys Pro Gly Gly Ala Ala Gly Ile
        355                 360                 365

gcc aag tgg cgt tgc gtt cta atg aag agg ata ccc gac tcc ggc tac 1152
Ala Lys Trp Arg Cys Val Leu Met Lys Arg Ile Pro Asp Ser Gly Tyr
    370                 375                 380

gat gag tac gat gat gac atc agt tcg aca act ccg gca ccc agc ggt 1200
Asp Glu Tyr Asp Asp Asp Ile Ser Ser Thr Thr Pro Ala Pro Ser Gly
385                 390                 395                 400

ggc gac tgt ctg cac aac agc agc agc tgc gag ccg ccg gtg agc atg 1248
Gly Asp Cys Leu His Asn Ser Ser Ser Cys Glu Pro Pro Val Ser Met
                405                 410                 415

gcc cac aag gta aac cag cgt ctg cgc aac ttt gag ccc acc tgg cat 1296
Ala His Lys Val Asn Gln Arg Leu Arg Asn Phe Glu Pro Thr Trp His
            420                 425                 430

ccc gcg aca cct gat ctg acg caa tgc cgc agc ctt tgg ctc aac aat 1344
Pro Ala Thr Pro Asp Leu Thr Gln Cys Arg Ser Leu Trp Leu Asn Asn
        435                 440                 445

ctg gaa atg cga gta aac cag cgg gac tcc tcc ttg atc tcc atc gcc 1392
Leu Glu Met Arg Val Asn Gln Arg Asp Ser Ser Leu Ile Ser Ile Ala
    450                 455                 460

aac gat atg tcc gaa gtg acc agt agc aaa acg ctc tac ggc ggc gac 1440
Asn Asp Met Ser Glu Val Thr Ser Ser Lys Thr Leu Tyr Gly Gly Asp
465                 470                 475                 480

atg ttg gtc acc acg aag att atc caa aca gtg tcc gag aag atg atg 1488
Met Leu Val Thr Thr Lys Ile Ile Gln Thr Val Ser Glu Lys Met Met
                485                 490                 495

cac gac aag gag acc ttc ccg gat cag cga cag cgc gag gct atg atc 1536
His Asp Lys Glu Thr Phe Pro Asp Gln Arg Gln Arg Glu Ala Met Ile
            500                 505                 510

atg gag ttg ttg cat tgt gtg gtc aaa acc ggc tcc aac ctg ctg gac 1584
Met Glu Leu Leu His Cys Val Val Lys Thr Gly Ser Asn Leu Leu Asp
        515                 520                 525

gaa tcg cag ctg tcc tcg tgg ttg gat ctc aat ccg gag gac caa atg 1632
Glu Ser Gln Leu Ser Ser Trp Leu Asp Leu Asn Pro Glu Asp Gln Met
    530                 535                 540

cgt gta gcc aca tcc ttg cta act ggc ctg gaa tac aat gcc ttt ctg 1680
Arg Val Ala Thr Ser Leu Leu Thr Gly Leu Glu Tyr Asn Ala Phe Leu
545                 550                 555                 560

ctg gcg gat acg atc atc agg gag cgc agc gtg gtg caa aaa gtc aaa 1728
Leu Ala Asp Thr Ile Ile Arg Glu Arg Ser Val Val Gln Lys Val Lys
                565                 570                 575

aat ata ttg ctc tcc gtt cga gtt ctg gaa acc aag act atc cag tcc 1776
Asn Ile Leu Leu Ser Val Arg Val Leu Glu Thr Lys Thr Ile Gln Ser
            580                 585                 590

agc gtg gtc ttc cca gat tcg gat cag tgg ccc ttg agt tcg gat cgt 1824
Ser Val Val Phe Pro Asp Ser Asp Gln Trp Pro Leu Ser Ser Asp Arg
        595                 600                 605

att gag ctg cca cga gct gct cta ata gat aat agt gaa ggc ggt ctg 1872
Ile Glu Leu Pro Arg Ala Ala Leu Ile Asp Asn Ser Glu Gly Gly Leu
    610                 615                 620

gtg cga att gta ttc gcc gcc ttc gat cgc ctg gaa tcc att cta aag 1920
Val Arg Ile Val Phe Ala Ala Phe Asp Arg Leu Glu Ser Ile Leu Lys
625                 630                 635                 640

ccc agc tat gat cac ttc gat ctc aag agc tcc cgc agt tac gcc atc 1968
Pro Ser Tyr Asp His Phe Asp Leu Lys Ser Ser Arg Ser Tyr Ala Ile
                645                 650                 655

ctg agc aac gac agc gat gtc aac gcg ggg gag atc caa cag cgc cta 2016
Leu Ser Asn Asp Ser Asp Val Asn Ala Gly Glu Ile Gln Gln Arg Leu
            660                 665                 670

cgc atc ctg aac agc aag gtg atc tcg gcc agc ttg ggc aag ggg cgt 2064
Arg Ile Leu Asn Ser Lys Val Ile Ser Ala Ser Leu Gly Lys Gly Arg
        675                 680                 685

cac ata caa ctc tcc cag ccc ata acc ctg aca ctg aaa cat ctg aag 2112
His Ile Gln Leu Ser Gln Pro Ile Thr Leu Thr Leu Lys His Leu Lys
    690                 695                 700

acc gag aat gta acg aat ccc acc tgc gtg ttc tgg aac tat att gac 2160
Thr Glu Asn Val Thr Asn Pro Thr Cys Val Phe Trp Asn Tyr Ile Asp
705                 710                 715                 720

cat gcg tgg tct gcc aac gga tgc agt ctg gag tcc act aac cgc acg 2208
His Ala Trp Ser Ala Asn Gly Cys Ser Leu Glu Ser Thr Asn Arg Thr
                725                 730                 735

cac agc gtc tgc agt tgc aac cac ctg aca aac ttt gcc ata cta atg 2256
His Ser Val Cys Ser Cys Asn His Leu Thr Asn Phe Ala Ile Leu Met
            740                 745                 750

gac gtt gtg gat gag cac cag cat tcg ttg ttc acc atg ttc gat gga 2304
Asp Val Val Asp Glu His Gln His Ser Leu Phe Thr Met Phe Asp Gly
        755                 760                 765

aac atg cgc ata ttc atc tac ata agc atc ggc atc tgc gtg gtc ttc 2352
Asn Met Arg Ile Phe Ile Tyr Ile Ser Ile Gly Ile Cys Val Val Phe
    770                 775                 780

ata gtt atc gcc ctg cta acg ctg aag ctg ttc aat ggg gtc ttt gtg 2400
Ile Val Ile Ala Leu Leu Thr Leu Lys Leu Phe Asn Gly Val Phe Val
785                 790                 795                 800

aag tcc gcg cgc acc tcg atc tat acc agc att tac ctt tgc ctc ctg 2448
Lys Ser Ala Arg Thr Ser Ile Tyr Thr Ser Ile Tyr Leu Cys Leu Leu
                805                 810                 815

gcc atc gag ctg ctc ttt ctc ctg ggc att gaa cag acc gaa aca agc 2496
Ala Ile Glu Leu Leu Phe Leu Leu Gly Ile Glu Gln Thr Glu Thr Ser
            820                 825                 830

att ttc tgc ggc ttc att act att ttc cta cac tgt gcc atc cta tcg 2544
Ile Phe Cys Gly Phe Ile Thr Ile Phe Leu His Cys Ala Ile Leu Ser
        835                 840                 845

ggc acc gcc tgg ttc tgt tac gaa gcc ttc cat tcg tac tca acg ctc    2592
Gly Thr Ala Trp Phe Cys Tyr Glu Ala Phe His Ser Tyr Ser Thr Leu
    850                 855                 860

acc tcg gac gag ctc ctg ctg gag gtg gac cag acg ccc aag gtg aac    2640
Thr Ser Asp Glu Leu Leu Leu Glu Val Asp Gln Thr Pro Lys Val Asn
865                 870                 875                 880

tgc tac tac ctc ttg tcc tac gga ctg tcg ctg agc gtg gtg gcc atc    2688
Cys Tyr Tyr Leu Leu Ser Tyr Gly Leu Ser Leu Ser Val Val Ala Ile
                885                 890                 895

tcg ctg gtc atc gat ccc agc acc tat acc caa aac gat tat tgc gtg    2736
Ser Leu Val Ile Asp Pro Ser Thr Tyr Thr Gln Asn Asp Tyr Cys Val
            900                 905                 910

ctg atg gag gcg aat gcc ttg ttt tat gcc acc ttt gta ata cca gtg    2784
Leu Met Glu Ala Asn Ala Leu Phe Tyr Ala Thr Phe Val Ile Pro Val
        915                 920                 925

ctt gtc ttc ttt gtg gct gcc att ggt tac aca ttc ctc tcc tgg att    2832
Leu Val Phe Phe Val Ala Ala Ile Gly Tyr Thr Phe Leu Ser Trp Ile
    930                 935                 940

ata atg tgc cgc aaa agt cgc acg ggt cta aag acc aag gaa cat act    2880
Ile Met Cys Arg Lys Ser Arg Thr Gly Leu Lys Thr Lys Glu His Thr
945                 950                 955                 960

cgc ctc gct agc gtg cgg ttc gac ata cgc tgc tcc ttt gtg ttc ctc    2928
Arg Leu Ala Ser Val Arg Phe Asp Ile Arg Cys Ser Phe Val Phe Leu
                965                 970                 975

ttg ctg ctc agc gct gtt tgg tgc tcg gcc tac ttc tat ttg cga gga    2976
Leu Leu Leu Ser Ala Val Trp Cys Ser Ala Tyr Phe Tyr Leu Arg Gly
            980                 985                 990

gcc aaa atg gac gat gac acg gct gat gtg tat gga tac tgc ttc atc    3024
Ala Lys Met Asp Asp Asp Thr Ala Asp Val Tyr Gly Tyr Cys Phe Ile
        995                 1000                1005

tgc ttc aac aca ttg ctg ggg ctc tat atc ttc gtg ttc cat tgc att    3072
Cys Phe Asn Thr Leu Leu Gly Leu Tyr Ile Phe Val Phe His Cys Ile
   1010                1015                1020

caa aac gaa aag atc cgg cgg gag tat cgg aag tat gtg aga cag cac    3120
Gln Asn Glu Lys Ile Arg Arg Glu Tyr Arg Lys Tyr Val Arg Gln His
1025                1030                1035                1040

gct tgg ctg ccc aag tgc ttg cgc tgc tcg aaa aca tca att tcc tcg   3168
Ala Trp Leu Pro Lys Cys Leu Arg Cys Ser Lys Thr Ser Ile Ser Ser
                1045                1050                1055

ggc att gtt acc ggc aat gga ccc aca gcc gga acc ctt tgc agc gtc   3216
Gly Ile Val Thr Gly Asn Gly Pro Thr Ala Gly Thr Leu Cys Ser Val
            1060                1065                1070

tcc acg tcc aag aag ccc aag ctg ccg tta gga gtg agc gaa gag gcg   3264
Ser Thr Ser Lys Lys Pro Lys Leu Pro Leu Gly Val Ser Glu Glu Ala
        1075                1080                1085

cat gac gat ccc cag cag caa cag cag aca cca gtg ccc atc aca gag   3312
His Asp Asp Pro Gln Gln Gln Gln Gln Thr Pro Val Pro Ile Thr Glu
    1090                1095                1100

gat gcc att atg gga gcc acc tct gat tgt gaa ctg aac gag gcc cag   3360
Asp Ala Ile Met Gly Ala Thr Ser Asp Cys Glu Leu Asn Glu Ala Gln
1105                1110                1115                1120

caa aga aga acc cta aaa agt ggc cta atg acg ggc aca cta cag gct   3408
Gln Arg Arg Thr Leu Lys Ser Gly Leu Met Thr Gly Thr Leu Gln Ala
                1125                1130                1135

cca ccg cag acc ctt ggt ggc cat gtt gtg ctc gaa aga ggt agc act   3456
Pro Pro Gln Thr Leu Gly Gly His Val Val Leu Glu Arg Gly Ser Thr
            1140                1145                1150

ctc cgc tcc act ggt cat gcc tca ccc acc agc tct gcc ggg tcc aca   3504
Leu Arg Ser Thr Gly His Ala Ser Pro Thr Ser Ser Ala Gly Ser Thr
        1155                1160                1165

cac ctg att ttt gcg cac aag caa caa caa caa cag cag caa cag gga   3552
His Leu Ile Phe Ala His Lys Gln Gln Gln Gln Gln Gln Gln Gln Gly
    1170                1175                1180

cct ttg ggc gag tct tac tac cat cag ccg gac tac tac agc tgg aag   3600
Pro Leu Gly Glu Ser Tyr Tyr His Gln Pro Asp Tyr Tyr Ser Trp Lys
1185                1190                1195                1200

caa cca tca act gga aca gga gga ttg aaa aca ccg cgg gag tac tac   3648
Gln Pro Ser Thr Gly Thr Gly Gly Leu Lys Thr Pro Arg Glu Tyr Tyr
                1205                1210                1215

aat aat gcg ggt gct gct gca tca tcg ccg aca ggc gca cga ggt att   3696
Asn Asn Ala Gly Ala Ala Ala Ser Ser Pro Thr Gly Ala Arg Gly Ile
            1220                1225                1230

cta ctg gac tca aaa gcc gaa cag cgg cca caa tgg caa aaa gaa gag 3744
Leu Leu Asp Ser Lys Ala Glu Gln Arg Pro Gln Trp Gln Lys Glu Glu
        1235                1240                1245

ggg cgc cgg agg agt tcc cgc ctc gcc tat cgc acg gcc gcc gcc tcc 3792
Gly Arg Arg Arg Ser Ser Arg Leu Ala Tyr Arg Thr Ala Ala Ala Ser
    1250                1255                1260

cag gtg ctt ttc tat cca tcg tac aag aag acc aag cct ggc cag cca 3840
Gln Val Leu Phe Tyr Pro Ser Tyr Lys Lys Thr Lys Pro Gly Gln Pro
1265                1270                1275                1280

aca ggc tat ccg caa tac gcg gag gcg ttg gac cca cca cta gcc act 3888
Thr Gly Tyr Pro Gln Tyr Ala Glu Ala Leu Asp Pro Pro Leu Ala Thr
                1285                1290                1295

ggc aat gcg gct gcc tac tac cag cag cag caa cag ttg cgt cgc cag 3936
Gly Asn Ala Ala Ala Tyr Tyr Gln Gln Gln Gln Gln Leu Arg Arg Gln
            1300                1305                1310

cag cta cat cag cag cag caa cag cag cag cag cag caa ctc tcc tcg 3984
Gln Leu His Gln Gln Gln Gln Gln Gln Gln Gln Gln Gln Leu Ser Ser
        1315                1320                1325

gac gag gag cag gcc gag caa cat gct cac ctg ttg cac ctg caa cga 4032
Asp Glu Glu Gln Ala Glu Gln His Ala His Leu Leu His Leu Gln Arg
    1330                1335                1340

cga gct ggt agc cag cag cag ctc cct gct cca ccg cca cac atg gcg 4080
Arg Ala Gly Ser Gln Gln Gln Leu Pro Ala Pro Pro Pro His Met Ala
1345                1350                1355                1360

cag tac cag cag gag ttt atg cag cgc cag tat aga aat aag cat tcc 4128
Gln Tyr Gln Gln Glu Phe Met Gln Arg Gln Tyr Arg Asn Lys His Ser
                1365                1370                1375

aac tgt gat ctg ggc atg ggc gat gcc tac tac aac caa ggc agc gtc 4176
Asn Cys Asp Leu Gly Met Gly Asp Ala Tyr Tyr Asn Gln Gly Ser Val
            1380                1385                1390

ggc ggc gcg gat ggt ggg ccg gtc tac gag gag atc ctc agc aac cgc 4224
Gly Gly Ala Asp Gly Gly Pro Val Tyr Glu Glu Ile Leu Ser Asn Arg
        1395                1400                1405

aac tcg gat gtg cag cat tac gag gtg ggt gac ttc gat gtg gac gag 4272
Asn Ser Asp Val Gln His Tyr Glu Val Gly Asp Phe Asp Val Asp Glu
    1410                1415                1420

gtg tac aac aat agc gtt ggc act ggc gtc ttc aac aac atg aga gcg    4320
Val Tyr Asn Asn Ser Val Gly Thr Gly Val Phe Asn Asn Met Arg Ala
1425                1430                1435                1440

gcg gtg gcc gcc ggc ggt agt                                         4341
Ala Val Ala Ala Gly Gly Ser
                1445

<210> 2
<211> 1447
<212> PRT
<213> Drosophila melanogaster

<400> 2
   Met Ile His Lys Leu Asn Gly Thr Phe Glu Ser Asn Phe His Glu Tyr
    1               5                  10                  15
   Asp Ser Lys Arg Lys Tyr Ile Arg Val Ser Lys Tyr Gln Thr Ala Tyr
               20                  25                  30
   Ala Cys Glu Gly Lys Lys Leu Thr Ile Glu Cys Asp Pro Gly Asp Val
           35                  40                  45
   Ile Asn Leu Ile Arg Ala Asn Tyr Gly Arg Phe Ser Ile Thr Ile Cys
       50                  55                  60
   Asn Asp His Gly Asn Val Glu Trp Ser Val Asn Cys Met Phe Pro Lys
   65                  70                  75                  80
   Ser Leu Ser Val Leu Asn Ser Arg Cys Ala His Lys Gln Ser Cys Gly
                   85                  90                  95
   Val Leu Ala Ala Thr Ser Met Phe Gly Asp Pro Cys Pro Gly Thr His
               100                 105                 110
   Lys Tyr Leu Glu Ala His Tyr Gln Cys Ile Ser Ala Ala Gln Thr Ser
           115                 120                 125
   Thr Thr Thr Asn Arg Pro Ser Pro Pro Pro Trp Val Leu Ser Asn Gly
       130                 135                 140
   Pro Pro Ile Phe Gly Asn Gly Ser Gly Leu Ile His Pro Pro Gly Val
   145                 150                 155                 160
   Gly Ala Gly Ala Pro Pro Pro Pro Arg Leu Pro Thr Leu Pro Gly Val
                   165                 170                 175
   Val Gly Ile Ser Gly Asn Pro Gly Leu Phe Asn Val Pro Pro Gln His
   180 185 190
   Thr Ala Val Thr His Ser Thr Pro Ser Ser Ser Thr Thr Ala Val Gly
   195 200 205
   Gly Gly Arg Leu Lys Gly Gly Ala Thr Ser Thr Thr Thr Thr Lys His
   210 215 220
   Pro Ala Gly Arg His Asp Gly Leu Pro Pro Pro Pro Gln Leu His His
   225 230 235 240
   His His Asn His His Gly Glu Asp Thr Ala Ser Pro Thr Lys Pro Ser
   245 250 255
   Ser Lys Leu Pro Ala Gly Gly Asn Ala Thr Ser Pro Ser Asn Thr Arg
   260 265 270
   Ile Leu Thr Gly Val Gly Gly Ser Gly Thr Asp Asp Gly Thr Leu Leu
   275 280 285
   Thr Thr Lys Ser Ser Pro Asn Arg Pro Pro Gly Thr Ala Ala Ser Gly
   290 295 300
   Ser Val Val Pro Gly Asn Gly Ser Val Val Arg Thr Ile Asn Asn Ile
   305 310 315 320
   Asn Leu Asn Ala Ala Gly Met Ser Gly Gly Asp Asp Glu Ser Lys Leu
   325 330 335
   Phe Cys Gly Pro Thr His Ala Arg Asn Leu Tyr Trp Asn Met Thr Arg
   340 345 350
   Val Gly Asp Val Asn Val Gln Pro Cys Pro Gly Gly Ala Ala Gly Ile
   355 360 365
   Ala Lys Trp Arg Cys Val Leu Met Lys Arg Ile Pro Asp Ser Gly Tyr
   370 375 380
   Asp Glu Tyr Asp Asp Asp Ile Ser Ser Thr Thr Pro Ala Pro Ser Gly
   385 390 395 400
   Gly Asp Cys Leu His Asn Ser Ser Ser Cys Glu Pro Pro Val Ser Met
   405 410 415
   Ala His Lys Val Asn Gln Arg Leu Arg Asn Phe Glu Pro Thr Trp His
   420 425 430
   Pro Ala Thr Pro Asp Leu Thr Gln Cys Arg Ser Leu Trp Leu Asn Asn
   435 440 445
   Leu Glu Met Arg Val Asn Gln Arg Asp Ser Ser Leu Ile Ser Ile Ala
   450 455 460
   Asn Asp Met Ser Glu Val Thr Ser Ser Lys Thr Leu Tyr Gly Gly Asp
   465 470 475 480
   Met Leu Val Thr Thr Lys Ile Ile Gln Thr Val Ser Glu Lys Met Met
   485 490 495
   His Asp Lys Glu Thr Phe Pro Asp Gln Arg Gln Arg Glu Ala Met Ile
   500 505 510
   Met Glu Leu Leu His Cys Val Val Lys Thr Gly Ser Asn Leu Leu Asp
   515 520 525
   Glu Ser Gln Leu Ser Ser Trp Leu Asp Leu Asn Pro Glu Asp Gln Met
   530 535 540
   Arg Val Ala Thr Ser Leu Leu Thr Gly Leu Glu Tyr Asn Ala Phe Leu
   545 550 555 560
   Leu Ala Asp Thr Ile Ile Arg Glu Arg Ser Val Val Gln Lys Val Lys
   565 570 575
   Asn Ile Leu Leu Ser Val Arg Val Leu Glu Thr Lys Thr Ile Gln Ser
   580 585 590
   Ser Val Val Phe Pro Asp Ser Asp Gln Trp Pro Leu Ser Ser Asp Arg
   595 600 605
   Ile Glu Leu Pro Arg Ala Ala Leu Ile Asp Asn Ser Glu Gly Gly Leu
   610 615 620
   Val Arg Ile Val Phe Ala Ala Phe Asp Arg Leu Glu Ser Ile Leu Lys
   625 630 635 640
   Pro Ser Tyr Asp His Phe Asp Leu Lys Ser Ser Arg Ser Tyr Ala Ile
   645 650 655
   Leu Ser Asn Asp Ser Asp Val Asn Ala Gly Glu Ile Gln Gln Arg Leu
   660 665 670
   Arg Ile Leu Asn Ser Lys Val Ile Ser Ala Ser Leu Gly Lys Gly Arg
   675 680 685
   His Ile Gln Leu Ser Gln Pro Ile Thr Leu Thr Leu Lys His Leu Lys
       690                 695                 700
   Thr Glu Asn Val Thr Asn Pro Thr Cys Val Phe Trp Asn Tyr Ile Asp
   705                 710                 715                 720
   His Ala Trp Ser Ala Asn Gly Cys Ser Leu Glu Ser Thr Asn Arg Thr
                   725                 730                 735
   His Ser Val Cys Ser Cys Asn His Leu Thr Asn Phe Ala Ile Leu Met
               740                 745                 750
   Asp Val Val Asp Glu His Gln His Ser Leu Phe Thr Met Phe Asp Gly
           755                 760                 765
   Asn Met Arg Ile Phe Ile Tyr Ile Ser Ile Gly Ile Cys Val Val Phe
       770                 775                 780
   Ile Val Ile Ala Leu Leu Thr Leu Lys Leu Phe Asn Gly Val Phe Val
   785                 790                 795                 800
   Lys Ser Ala Arg Thr Ser Ile Tyr Thr Ser Ile Tyr Leu Cys Leu Leu
                   805                 810                 815
   Ala Ile Glu Leu Leu Phe Leu Leu Gly Ile Glu Gln Thr Glu Thr Ser
               820                 825                 830
   Ile Phe Cys Gly Phe Ile Thr Ile Phe Leu His Cys Ala Ile Leu Ser
           835                 840                 845
   Gly Thr Ala Trp Phe Cys Tyr Glu Ala Phe His Ser Tyr Ser Thr Leu
       850                 855                 860
   Thr Ser Asp Glu Leu Leu Leu Glu Val Asp Gln Thr Pro Lys Val Asn
   865                 870                 875                 880
   Cys Tyr Tyr Leu Leu Ser Tyr Gly Leu Ser Leu Ser Val Val Ala Ile
                   885                 890                 895
   Ser Leu Val Ile Asp Pro Ser Thr Tyr Thr Gln Asn Asp Tyr Cys Val
               900                 905                 910
   Leu Met Glu Ala Asn Ala Leu Phe Tyr Ala Thr Phe Val Ile Pro Val
           915                 920                 925
   Leu Val Phe Phe Val Ala Ala Ile Gly Tyr Thr Phe Leu Ser Trp Ile
       930                 935                 940
   Ile Met Cys Arg Lys Ser Arg Thr Gly Leu Lys Thr Lys Glu His Thr
   945                 950                 955                 960
   Arg Leu Ala Ser Val Arg Phe Asp Ile Arg Cys Ser Phe Val Phe Leu
                   965                 970                 975
   Leu Leu Leu Ser Ala Val Trp Cys Ser Ala Tyr Phe Tyr Leu Arg Gly
               980                 985                 990
   Ala Lys Met Asp Asp Asp Thr Ala Asp Val Tyr Gly Tyr Cys Phe Ile
           995                 1000                1005
   Cys Phe Asn Thr Leu Leu Gly Leu Tyr Ile Phe Val Phe His Cys Ile
      1010                1015                1020
   Gln Asn Glu Lys Ile Arg Arg Glu Tyr Arg Lys Tyr Val Arg Gln His
   025                 1030                1035                1040
   Ala Trp Leu Pro Lys Cys Leu Arg Cys Ser Lys Thr Ser Ile Ser Ser
                   1045                1050                1055
   Gly Ile Val Thr Gly Asn Gly Pro Thr Ala Gly Thr Leu Cys Ser Val
               1060                1065                1070
   Ser Thr Ser Lys Lys Pro Lys Leu Pro Leu Gly Val Ser Glu Glu Ala
           1075                1080                1085
   His Asp Asp Pro Gln Gln Gln Gln Gln Thr Pro Val Pro Ile Thr Glu
      1090                1095                1100
   Asp Ala Ile Met Gly Ala Thr Ser Asp Cys Glu Leu Asn Glu Ala Gln
   105                 1110                1115                1120
   Gln Arg Arg Thr Leu Lys Ser Gly Leu Met Thr Gly Thr Leu Gln Ala
                   1125                1130                1135
   Pro Pro Gln Thr Leu Gly Gly His Val Val Leu Glu Arg Gly Ser Thr
               1140                1145                1150
   Leu Arg Ser Thr Gly His Ala Ser Pro Thr Ser Ser Ala Gly Ser Thr
           1155                1160                1165
   His Leu Ile Phe Ala His Lys Gln Gln Gln Gln Gln Gln Gln Gln Gly
      1170                1175                1180
   Pro Leu Gly Glu Ser Tyr Tyr His Gln Pro Asp Tyr Tyr Ser Trp Lys
   185                 1190                1195                1200
   Gln Pro Ser Thr Gly Thr Gly Gly Leu Lys Thr Pro Arg Glu Tyr Tyr
   1205 1210 1215
   Asn Asn Ala Gly Ala Ala Ala Ser Ser Pro Thr Gly Ala Arg Gly Ile
   1220 1225 1230
   Leu Leu Asp Ser Lys Ala Glu Gln Arg Pro Gln Trp Gln Lys Glu Glu
   1235 1240 1245
   Gly Arg Arg Arg Ser Ser Arg Leu Ala Tyr Arg Thr Ala Ala Ala Ser
   1250 1255 1260
   Gln Val Leu Phe Tyr Pro Ser Tyr Lys Lys Thr Lys Pro Gly Gln Pro
   265 1270 1275 1280
   Thr Gly Tyr Pro Gln Tyr Ala Glu Ala Leu Asp Pro Pro Leu Ala Thr
   1285 1290 1295
   Gly Asn Ala Ala Ala Tyr Tyr Gln Gln Gln Gln Gln Leu Arg Arg Gln
   1300 1305 1310
   Gln Leu His Gln Gln Gln Gln Gln Gln Gln Gln Gln Gln Leu Ser Ser
   1315 1320 1325
   Asp Glu Glu Gln Ala Glu Gln His Ala His Leu Leu His Leu Gln Arg
   1330 1335 1340
   Arg Ala Gly Ser Gln Gln Gln Leu Pro Ala Pro Pro Pro His Met Ala
   345 1350 1355 1360
   Gln Tyr Gln Gln Glu Phe Met Gln Arg Gln Tyr Arg Asn Lys His Ser
   1365 1370 1375
   Asn Cys Asp Leu Gly Met Gly Asp Ala Tyr Tyr Asn Gln Gly Ser Val
   1380 1385 1390
   Gly Gly Ala Asp Gly Gly Pro Val Tyr Glu Glu Ile Leu Ser Asn Arg
   1395 1400 1405
   Asn Ser Asp Val Gln His Tyr Glu Val Gly Asp Phe Asp Val Asp Glu
   1410 1415 1420
   Val Tyr Asn Asn Ser Val Gly Thr Gly Val Phe Asn Asn Met Arg Ala
   425 1430 1435 1440
   Ala Val Ala Ala Gly Gly Ser
   1445

<210> 3
<211> 4065
<212> DNA
<213> Drosophila melanogaster

<220>
<221> CDS
<222> (1)..(4062)

<400> 3
ata cat aag ttg aat ggt act ttt gag tcc aac ttt cat gaa tat gac     48
Ile His Lys Leu Asn Gly Thr Phe Glu Ser Asn Phe His Glu Tyr Asp
1               5                   10                  15

tcc aaa cgg aaa tac ata aga gtg tcc aag tac caa acc gcc tac gcc     96
Ser Lys Arg Lys Tyr Ile Arg Val Ser Lys Tyr Gln Thr Ala Tyr Ala
            20                  25                  30

tgc gaa ggt aag aaa ctg acc atc gag tgc gat ccc ggc gat gtg atc     144
Cys Glu Gly Lys Lys Leu Thr Ile Glu Cys Asp Pro Gly Asp Val Ile
        35                  40                  45

aac ctc att cgg gcc aac tat ggc cgc ttc tcg att acc atc tgc aat     192
Asn Leu Ile Arg Ala Asn Tyr Gly Arg Phe Ser Ile Thr Ile Cys Asn
    50                  55                  60

gac cac ggg aat gtg gag tgg agt gtt aac tgc atg ttt ccc aag tca     240
Asp His Gly Asn Val Glu Trp Ser Val Asn Cys Met Phe Pro Lys Ser
65                  70                  75                  80

ctc agc gta ctg aac tca aga tgt gcc cac aag cag agc tgc ggc gtg     288
Leu Ser Val Leu Asn Ser Arg Cys Ala His Lys Gln Ser Cys Gly Val
                85                  90                  95

ttg gca gcc acg agc atg ttc ggg gat ccc tgt ccc ggt acc cac aag     336
Leu Ala Ala Thr Ser Met Phe Gly Asp Pro Cys Pro Gly Thr His Lys
            100                 105                 110

tat ctg gag gca cac tac cag tgc ata agt gca gcc caa act tcg acg     384
Tyr Leu Glu Ala His Tyr Gln Cys Ile Ser Ala Ala Gln Thr Ser Thr
        115                 120                 125

acg acc aac agg ccc agt ccg ccg cca tgg gtg ctg agc aat ggt ccg     432
Thr Thr Asn Arg Pro Ser Pro Pro Pro Trp Val Leu Ser Asn Gly Pro
    130                 135                 140

ccg atc ttt ggc aac ggc agt gga ctg atc cat ccg ccc ggg gtt gga     480
Pro Ile Phe Gly Asn Gly Ser Gly Leu Ile His Pro Pro Gly Val Gly
145                 150                 155                 160

gcg ggt gcg ccg ccc ccg ccg aga ctt ccc aca ctt ccc gga gtg gtg     528
Ala Gly Ala Pro Pro Pro Pro Arg Leu Pro Thr Leu Pro Gly Val Val
                165                 170                 175

gga atc agt ggg aat ccc ggc ctg ttc aac gta cca ccg caa cac acc     576
Gly Ile Ser Gly Asn Pro Gly Leu Phe Asn Val Pro Pro Gln His Thr
            180                 185                 190

gcc gtc acg cac tcc acg ccc tcg agc agc acg aca gcc gtg ggc ggt     624
Ala Val Thr His Ser Thr Pro Ser Ser Ser Thr Thr Ala Val Gly Gly
        195                 200                 205

gga cgt ttg aag ggt ggg gcc acc tcc acg acg acc acc aag cat ccg     672
Gly Arg Leu Lys Gly Gly Ala Thr Ser Thr Thr Thr Thr Lys His Pro
    210                 215                 220

gct ggc cgc cat gat ggt ctg cca ccg ccg ccg caa ctg cac cac cac     720
Ala Gly Arg His Asp Gly Leu Pro Pro Pro Pro Gln Leu His His His
225                 230                 235                 240

cac aac cac cac ggt gaa gac act gcc tca ccc acc aag ccg agc agc     768
His Asn His His Gly Glu Asp Thr Ala Ser Pro Thr Lys Pro Ser Ser
                245                 250                 255

aag ctg ccg gct ggc ggt aat gcc act tca cca tcc aac acg agg ata     816
Lys Leu Pro Ala Gly Gly Asn Ala Thr Ser Pro Ser Asn Thr Arg Ile
            260                 265                 270

ctc acg ggc gtc gga ggt tcc gga act gat gac gga acc cta ctg acc     864
Leu Thr Gly Val Gly Gly Ser Gly Thr Asp Asp Gly Thr Leu Leu Thr
        275                 280                 285

aca aag agc tca ccc aac cgc cca ccg ggc act gcg gcc agt gga tcc     912
Thr Lys Ser Ser Pro Asn Arg Pro Pro Gly Thr Ala Ala Ser Gly Ser
    290                 295                 300

gtt gtc ccc ggg aac ggc agc gtg gtg cgc acc atc aac aat att aat     960
Val Val Pro Gly Asn Gly Ser Val Val Arg Thr Ile Asn Asn Ile Asn
305                 310                 315                 320

ttg aac gca gcc ggg atg tcc gga ggc gat gat gag tcc aag ttg ttt     1008
Leu Asn Ala Ala Gly Met Ser Gly Gly Asp Asp Glu Ser Lys Leu Phe
                325                 330                 335

tgc ggc ccc act cat gcc cgc aat ttg tac tgg aac atg act cga gtg    1056
Cys Gly Pro Thr His Ala Arg Asn Leu Tyr Trp Asn Met Thr Arg Val
            340                 345                 350

ggt gat gtg aat gtt cag ccc tgt cct ggc gga gca gcc ggc atc gcc    1104
Gly Asp Val Asn Val Gln Pro Cys Pro Gly Gly Ala Ala Gly Ile Ala
        355                 360                 365

aag tgg cgt tgc gtt cta atg aag agg ata ccc gac tcc ggc tac gat    1152
Lys Trp Arg Cys Val Leu Met Lys Arg Ile Pro Asp Ser Gly Tyr Asp
    370                 375                 380

gag tac gat gat gac atc agt tcg aca act ccg gca ccc agc ggt ggc    1200
Glu Tyr Asp Asp Asp Ile Ser Ser Thr Thr Pro Ala Pro Ser Gly Gly
385                 390                 395                 400

gac tgt ctg cac aac agc agc agc tgc gag ccg ccg gtg agc atg gcc    1248
Asp Cys Leu His Asn Ser Ser Ser Cys Glu Pro Pro Val Ser Met Ala
                405                 410                 415

cac aag gta aac cag cgt ctg cgc aac ttt gag ccc acc tgg cat ccc    1296
His Lys Val Asn Gln Arg Leu Arg Asn Phe Glu Pro Thr Trp His Pro
            420                 425                 430

gcg aca cct gat ctg acg caa tgc cgc agc ctt tgg ctc aac aat ctg    1344
Ala Thr Pro Asp Leu Thr Gln Cys Arg Ser Leu Trp Leu Asn Asn Leu
        435                 440                 445

gaa atg cga gta aac cag cgg gac tcc tcc ttg atc tcc atc gcc aac    1392
Glu Met Arg Val Asn Gln Arg Asp Ser Ser Leu Ile Ser Ile Ala Asn
    450                 455                 460

gat atg tcc gaa gtg acc agt agc aaa acg ctc tac ggc ggc gac atg    1440
Asp Met Ser Glu Val Thr Ser Ser Lys Thr Leu Tyr Gly Gly Asp Met
465                 470                 475                 480

ttg gtc acc acg aag att atc caa aca gtg tcc gag aag atg atg cac    1488
Leu Val Thr Thr Lys Ile Ile Gln Thr Val Ser Glu Lys Met Met His
                485                 490                 495

gac aag gag acc ttc ccg gat cag cga cag cgc gag gct atg atc atg    1536
Asp Lys Glu Thr Phe Pro Asp Gln Arg Gln Arg Glu Ala Met Ile Met
            500                 505                 510

gag ttg ttg cat tgt gtg gtc aaa acc ggc tcc aac ctg ctg gac gaa    1584
Glu Leu Leu His Cys Val Val Lys Thr Gly Ser Asn Leu Leu Asp Glu
        515                 520                 525

tcg cag ctg tcc tcg tgg ttg gat ctc aat ccg gag gac caa atg cgt 1632
Ser Gln Leu Ser Ser Trp Leu Asp Leu Asn Pro Glu Asp Gln Met Arg
    530                 535                 540

gta gcc aca tcc ttg cta act ggc ctg gaa tac aat gcc ttt ctg ctg 1680
Val Ala Thr Ser Leu Leu Thr Gly Leu Glu Tyr Asn Ala Phe Leu Leu
545                 550                 555                 560

gcg gat acg atc atc agg gag cgc agc gtg gtg caa aaa gtc aaa aat 1728
Ala Asp Thr Ile Ile Arg Glu Arg Ser Val Val Gln Lys Val Lys Asn
                565                 570                 575

ata ttg ctc tcc gtt cga gtt ctg gaa acc aag act atc cag tcc agc 1776
Ile Leu Leu Ser Val Arg Val Leu Glu Thr Lys Thr Ile Gln Ser Ser
            580                 585                 590

gtg gtc ttc cca gat tcg gat cag tgg ccc ttg agt tcg gat cgt att 1824
Val Val Phe Pro Asp Ser Asp Gln Trp Pro Leu Ser Ser Asp Arg Ile
        595                 600                 605

gag ctg cca cga gct gct cta ata gat aat agt gaa ggc ggt ctg gtg 1872
Glu Leu Pro Arg Ala Ala Leu Ile Asp Asn Ser Glu Gly Gly Leu Val
    610                 615                 620

cga att gta ttc gcc gcc ttc gat cgc ctg gaa tcc att cta aag ccc 1920
Arg Ile Val Phe Ala Ala Phe Asp Arg Leu Glu Ser Ile Leu Lys Pro
625                 630                 635                 640

agc tat gat cac ttc gat ctc aag agc tcc cgc agt tac gcc atc ctg 1968
Ser Tyr Asp His Phe Asp Leu Lys Ser Ser Arg Ser Tyr Ala Ile Leu
                645                 650                 655

agc aac gac agc gat gtc aac gcg ggg gag atc caa cag cgc cta cgc 2016
Ser Asn Asp Ser Asp Val Asn Ala Gly Glu Ile Gln Gln Arg Leu Arg
            660                 665                 670

atc ctg aac agc aag gtg atc tcg gcc agc ttg ggc aag ggg cgt cac 2064
Ile Leu Asn Ser Lys Val Ile Ser Ala Ser Leu Gly Lys Gly Arg His
        675                 680                 685

ata caa ctc tcc cag ccc ata acc ctg aca ctg aaa cat ctg aag acc 2112
Ile Gln Leu Ser Gln Pro Ile Thr Leu Thr Leu Lys His Leu Lys Thr
    690                 695                 700

gag aat gta acg aat ccc acc tgc gtg ttc tgg aac tat att gac cat 2160
Glu Asn Val Thr Asn Pro Thr Cys Val Phe Trp Asn Tyr Ile Asp His
705                 710                 715                 720

gcg tgg tct gcc aac gga tgc agt ctg gag tcc act aac cgc acg cac    2208
Ala Trp Ser Ala Asn Gly Cys Ser Leu Glu Ser Thr Asn Arg Thr His
                725                 730                 735

agc gtc tgc agt tgc aac cac ctg aca aac ttt gcc ata cta atg gac    2256
Ser Val Cys Ser Cys Asn His Leu Thr Asn Phe Ala Ile Leu Met Asp
            740                 745                 750

gtt gtg gat gag cac cag cat tcg ttg ttc acc atg ttc gat gga aac    2304
Val Val Asp Glu His Gln His Ser Leu Phe Thr Met Phe Asp Gly Asn
        755                 760                 765

atg cgc ata ttc atc tac ata agc atc ggc atc tgc gtg gtc ttc ata    2352
Met Arg Ile Phe Ile Tyr Ile Ser Ile Gly Ile Cys Val Val Phe Ile
    770                 775                 780

gtt atc gcc ctg cta acg ctg aag ctg ttc aat ggg gtc ttt gtg aag    2400
Val Ile Ala Leu Leu Thr Leu Lys Leu Phe Asn Gly Val Phe Val Lys
785                 790                 795                 800

gta aga aac ggc tcc aat ccc ttg ccg cat cag cgg tcg ggc agc aga    2448
Val Arg Asn Gly Ser Asn Pro Leu Pro His Gln Arg Ser Gly Ser Arg
                805                 810                 815

cgc cag caa aac aat att cgc gac cag acc cac gag tcc ttg acc ctg    2496
Arg Gln Gln Asn Asn Ile Arg Asp Gln Thr His Glu Ser Leu Thr Leu
            820                 825                 830

acc acg cca acc agt cag tcc aat gtg ccg ccg ccc agt cat ggg aac    2544
Thr Thr Pro Thr Ser Gln Ser Asn Val Pro Pro Pro Ser His Gly Asn
        835                 840                 845

acg aac ttt atc caa cac aat tcc atc cgc aac tca cac cgc aac aat    2592
Thr Asn Phe Ile Gln His Asn Ser Ile Arg Asn Ser His Arg Asn Asn
    850                 855                 860

ctg aac tac aat gtc caa cag cag cag cag caa cag caa caa caa gtt    2640
Leu Asn Tyr Asn Val Gln Gln Gln Gln Gln Gln Gln Gln Gln Gln Val
865                 870                 875                 880

gtt gcg gca gct gct gct gct gtt gtg ctg tcc aat cag ccg cag cga    2688
Val Ala Ala Ala Ala Ala Ala Val Val Leu Ser Asn Gln Pro Gln Arg
                885                 890                 895

aat atg caa cat gcc atg aac aac ttg aat ctg aat ctg cat cag cac    2736
Asn Met Gln His Ala Met Asn Asn Leu Asn Leu Asn Leu His Gln His
            900                 905                 910

ggt cag cag acg gct gct gct gca gct gct gct gcc tca ata gcc gct   2784
Gly Gln Gln Thr Ala Ala Ala Ala Ala Ala Ala Ala Ser Ile Ala Ala
        915                 920                 925

gca ctg cag caa cat gca gtt cag gcc agc aac gcc agc aat aac ctc   2832
Ala Leu Gln Gln His Ala Val Gln Ala Ser Asn Ala Ser Asn Asn Leu
    930                 935                 940

aac atc agc cac aac tat ctg cag cag cag cat gtc cag cag cag cag   2880
Asn Ile Ser His Asn Tyr Leu Gln Gln Gln His Val Gln Gln Gln Gln
945                 950                 955                 960

cag cag cag cgt ggc cag cag ccg cag cca cat ccg cac cgc aat cac   2928
Gln Gln Gln Arg Gly Gln Gln Pro Gln Pro His Pro His Arg Asn His
                965                 970                 975

aac ctt aat gtg gac ggc aat gga ttg gat aac acg aac aac atc att   2976
Asn Leu Asn Val Asp Gly Asn Gly Leu Asp Asn Thr Asn Asn Ile Ile
            980                 985                 990

atg cag gcc aac atg gac gac tta caa tat aag tgg tgg tgg tgt ccg   3024
Met Gln Ala Asn Met Asp Asp Leu Gln Tyr Lys Trp Trp Trp Cys Pro
        995                 1000                1005

cgc gca cct cga tct ata cca gca ttt acc ttt gcc tcc tgg cca tcg   3072
Arg Ala Pro Arg Ser Ile Pro Ala Phe Thr Phe Ala Ser Trp Pro Ser
    1010                1015                1020

agc tgc tct ttc tcc tgg gca ttg aac aga ccg aaa caa gca ttc tgc   3120
Ser Cys Ser Phe Ser Trp Ala Leu Asn Arg Pro Lys Gln Ala Phe Cys
1025                1030                1035                1040

ggc ttc att act att ttc cta cac tgt gcc atc cta tcg ggc acc gcc   3168
Gly Phe Ile Thr Ile Phe Leu His Cys Ala Ile Leu Ser Gly Thr Ala
                1045                1050                1055

tgg ttc tgt tac gaa gcc ttc cat tcg tac tca acg ctc acc tcg gac   3216
Trp Phe Cys Tyr Glu Ala Phe His Ser Tyr Ser Thr Leu Thr Ser Asp
            1060                1065                1070

gag ctc ctg ctg gag gtg gac cag acg ccc aag gtg aac tgc tac tac   3264
Glu Leu Leu Leu Glu Val Asp Gln Thr Pro Lys Val Asn Cys Tyr Tyr
        1075                1080                1085

ctc ttg tcc tac gga ctg tcg ctg agc gtg gtg gcc atc tcg ctg gtc   3312
Leu Leu Ser Tyr Gly Leu Ser Leu Ser Val Val Ala Ile Ser Leu Val
    1090                1095                1100

atc gat ccc agc acc tat acc caa aac gat tat tgc gtg ctg atg gag 3360
Ile Asp Pro Ser Thr Tyr Thr Gln Asn Asp Tyr Cys Val Leu Met Glu
1105                1110                1115                1120

gcg aat gcc ttg ttt tat gcc acc ttt gta ata cca gtg ctt gtc ttc 3408
Ala Asn Ala Leu Phe Tyr Ala Thr Phe Val Ile Pro Val Leu Val Phe
                1125                1130                1135

ttt gtg gct gcc att ggt tac aca ttc ctc tcc tgg att ata atg tgc 3456
Phe Val Ala Ala Ile Gly Tyr Thr Phe Leu Ser Trp Ile Ile Met Cys
            1140                1145                1150

cgc aaa agt cgc acg ggt cta aag acc aag gaa cat act cgc ctc gct 3504
Arg Lys Ser Arg Thr Gly Leu Lys Thr Lys Glu His Thr Arg Leu Ala
        1155                1160                1165

agc gtg cgg ttc gac ata cgc tgc tcc ttt gtg ttc ctc ttg ctg ctc 3552
Ser Val Arg Phe Asp Ile Arg Cys Ser Phe Val Phe Leu Leu Leu Leu
    1170                1175                1180

agc gct gtt tgg tgc tcg gcc tac ttc tat ttg cga gga gcc aaa atg 3600
Ser Ala Val Trp Cys Ser Ala Tyr Phe Tyr Leu Arg Gly Ala Lys Met
1185                1190                1195                1200

gac gat gac acg gct gat gtg tat gga tac tgc ttc atc tgc ttc aac 3648
Asp Asp Asp Thr Ala Asp Val Tyr Gly Tyr Cys Phe Ile Cys Phe Asn
                1205                1210                1215

aca ttg ctg ggg ctc tat atc ttc gtg ttc cat tgc att caa aac gaa 3696
Thr Leu Leu Gly Leu Tyr Ile Phe Val Phe His Cys Ile Gln Asn Glu
            1220                1225                1230

aag atc cgg cgg gag tat cgg aag tat gtg aga cag cac gct tgg ctg 3744
Lys Ile Arg Arg Glu Tyr Arg Lys Tyr Val Arg Gln His Ala Trp Leu
        1235                1240                1245

ccc aag tgc ttg cgc tgc tcg aaa aca tca att tcc tcg ggc att gtt 3792
Pro Lys Cys Leu Arg Cys Ser Lys Thr Ser Ile Ser Ser Gly Ile Val
    1250                1255                1260

acc ggc aat gga ccc aca gcc gga acc ctt tgc agc gtc tcc acg tcc 3840
Thr Gly Asn Gly Pro Thr Ala Gly Thr Leu Cys Ser Val Ser Thr Ser
1265                1270                1275                1280

aag aag ccc aag ctg ccg tta gga gtg agc gaa gag gcg cat gac gat 3888
Lys Lys Pro Lys Leu Pro Leu Gly Val Ser Glu Glu Ala His Asp Asp
                1285                1290                1295

ccc cag cag caa cag cag aca cca gtg ccc atc aca gag gat gcc att    3936
Pro Gln Gln Gln Gln Gln Thr Pro Val Pro Ile Thr Glu Asp Ala Ile
            1300                1305                1310

atg gga gcc acc tct gat tgt gaa ctg aac gag gcc cag caa aga aga    3984
Met Gly Ala Thr Ser Asp Cys Glu Leu Asn Glu Ala Gln Gln Arg Arg
        1315                1320                1325

acc cta aaa agt ggc cta atg acg ggc aca cta cag gct cca ccg cag    4032
Thr Leu Lys Ser Gly Leu Met Thr Gly Thr Leu Gln Ala Pro Pro Gln
    1330                1335                1340

acc ctt ggt ggc cat gtt gtg ctc gaa aga gnn                        4065
Thr Leu Gly Gly His Val Val Leu Glu Arg
1345                1350

<210> 4
<211> 1354
<212> PRT
<213> Drosophila melanogaster

<400> 4
Ile His Lys Leu Asn Gly Thr Phe Glu Ser Asn Phe His Glu Tyr Asp
  1               5                  10                  15

Ser Lys Arg Lys Tyr Ile Arg Val Ser Lys Tyr Gln Thr Ala Tyr Ala
             20                  25                  30

Cys Glu Gly Lys Lys Leu Thr Ile Glu Cys Asp Pro Gly Asp Val Ile
         35                  40                  45

Asn Leu Ile Arg Ala Asn Tyr Gly Arg Phe Ser Ile Thr Ile Cys Asn
     50                  55                  60

Asp His Gly Asn Val Glu Trp Ser Val Asn Cys Met Phe Pro Lys Ser
 65                  70                  75                  80

Leu Ser Val Leu Asn Ser Arg Cys Ala His Lys Gln Ser Cys Gly Val
                 85                  90                  95

Leu Ala Ala Thr Ser Met Phe Gly Asp Pro Cys Pro Gly Thr His Lys
            100                 105                 110

Tyr Leu Glu Ala His Tyr Gln Cys Ile Ser Ala Ala Gln Thr Ser Thr
        115                 120                 125

Thr Thr Asn Arg Pro Ser Pro Pro Pro Trp Val Leu Ser Asn Gly Pro

130                 135                 140

Pro Ile Phe Gly Asn Gly Ser Gly Leu Ile His Pro Pro Gly Val Gly
145                 150                 155                 160

Ala Gly Ala Pro Pro Pro Pro Arg Leu Pro Thr Leu Pro Gly Val Val
                165                 170                 175

Gly Ile Ser Gly Asn Pro Gly Leu Phe Asn Val Pro Pro Gln His Thr
            180                 185                 190

Ala Val Thr His Ser Thr Pro Ser Ser Ser Thr Thr Ala Val Gly Gly
        195                 200                 205

Gly Arg Leu Lys Gly Gly Ala Thr Ser Thr Thr Thr Thr Lys His Pro
    210                 215                 220

Ala Gly Arg His Asp Gly Leu Pro Pro Pro Pro Gln Leu His His His
225                 230                 235                 240

His Asn His His Gly Glu Asp Thr Ala Ser Pro Thr Lys Pro Ser Ser
                245                 250                 255

Lys Leu Pro Ala Gly Gly Asn Ala Thr Ser Pro Ser Asn Thr Arg Ile
            260                 265                 270

Leu Thr Gly Val Gly Gly Ser Gly Thr Asp Asp Gly Thr Leu Leu Thr
        275                 280                 285

Thr Lys Ser Ser Pro Asn Arg Pro Pro Gly Thr Ala Ala Ser Gly Ser
    290                 295                 300

Val Val Pro Gly Asn Gly Ser Val Val Arg Thr Ile Asn Asn Ile Asn
305                 310                 315                 320

Leu Asn Ala Ala Gly Met Ser Gly Gly Asp Asp Glu Ser Lys Leu Phe
                325                 330                 335

Cys Gly Pro Thr His Ala Arg Asn Leu Tyr Trp Asn Met Thr Arg Val
            340                 345                 350

Gly Asp Val Asn Val Gln Pro Cys Pro Gly Gly Ala Ala Gly Ile Ala
        355                 360                 365

Lys Trp Arg Cys Val Leu Met Lys Arg Ile Pro Asp Ser Gly Tyr Asp
    370                 375                 380

Glu Tyr Asp Asp Asp Ile Ser Ser Thr Thr Pro Ala Pro Ser Gly Gly

385                 390                 395                 400

Asp Cys Leu His Asn Ser Ser Ser Cys Glu Pro Pro Val Ser Met Ala
                405                 410                 415

His Lys Val Asn Gln Arg Leu Arg Asn Phe Glu Pro Thr Trp His Pro
            420                 425                 430

Ala Thr Pro Asp Leu Thr Gln Cys Arg Ser Leu Trp Leu Asn Asn Leu
        435                 440                 445

Glu Met Arg Val Asn Gln Arg Asp Ser Ser Leu Ile Ser Ile Ala Asn
    450                 455                 460

Asp Met Ser Glu Val Thr Ser Ser Lys Thr Leu Tyr Gly Gly Asp Met
465                 470                 475                 480

Leu Val Thr Thr Lys Ile Ile Gln Thr Val Ser Glu Lys Met Met His
                485                 490                 495

Asp Lys Glu Thr Phe Pro Asp Gln Arg Gln Arg Glu Ala Met Ile Met
            500                 505                 510

Glu Leu Leu His Cys Val Val Lys Thr Gly Ser Asn Leu Leu Asp Glu
        515                 520                 525

Ser Gln Leu Ser Ser Trp Leu Asp Leu Asn Pro Glu Asp Gln Met Arg
    530                 535                 540

Val Ala Thr Ser Leu Leu Thr Gly Leu Glu Tyr Asn Ala Phe Leu Leu
545                 550                 555                 560

Ala Asp Thr Ile Ile Arg Glu Arg Ser Val Val Gln Lys Val Lys Asn
                565                 570                 575

Ile Leu Leu Ser Val Arg Val Leu Glu Thr Lys Thr Ile Gln Ser Ser
            580                 585                 590

Val Val Phe Pro Asp Ser Asp Gln Trp Pro Leu Ser Ser Asp Arg Ile
        595                 600                 605

Glu Leu Pro Arg Ala Ala Leu Ile Asp Asn Ser Glu Gly Gly Leu Val
    610                 615                 620

Arg Ile Val Phe Ala Ala Phe Asp Arg Leu Glu Ser Ile Leu Lys Pro
625                 630                 635                 640

Ser Tyr Asp His Phe Asp Leu Lys Ser Ser Arg Ser Tyr Ala Ile Leu

                645                 650                 655

Ser Asn Asp Ser Asp Val Asn Ala Gly Glu Ile Gln Gln Arg Leu Arg
            660                 665                 670

Ile Leu Asn Ser Lys Val Ile Ser Ala Ser Leu Gly Lys Gly Arg His
        675                 680                 685

Ile Gln Leu Ser Gln Pro Ile Thr Leu Thr Leu Lys His Leu Lys Thr
    690                 695                 700

Glu Asn Val Thr Asn Pro Thr Cys Val Phe Trp Asn Tyr Ile Asp His
705                 710                 715                 720

Ala Trp Ser Ala Asn Gly Cys Ser Leu Glu Ser Thr Asn Arg Thr His
                725                 730                 735

Ser Val Cys Ser Cys Asn His Leu Thr Asn Phe Ala Ile Leu Met Asp
            740                 745                 750

Val Val Asp Glu His Gln His Ser Leu Phe Thr Met Phe Asp Gly Asn
        755                 760                 765

Met Arg Ile Phe Ile Tyr Ile Ser Ile Gly Ile Cys Val Val Phe Ile
    770                 775                 780

Val Ile Ala Leu Leu Thr Leu Lys Leu Phe Asn Gly Val Phe Val Lys
785                 790                 795                 800

Val Arg Asn Gly Ser Asn Pro Leu Pro His Gln Arg Ser Gly Ser Arg
                805                 810                 815

Arg Gln Gln Asn Asn Ile Arg Asp Gln Thr His Glu Ser Leu Thr Leu
            820                 825                 830

Thr Thr Pro Thr Ser Gln Ser Asn Val Pro Pro Pro Ser His Gly Asn
        835                 840                 845

Thr Asn Phe Ile Gln His Asn Ser Ile Arg Asn Ser His Arg Asn Asn
    850                 855                 860

Leu Asn Tyr Asn Val Gln Gln Gln Gln Gln Gln Gln Gln Gln Gln Val
865                 870                 875                 880

Val Ala Ala Ala Ala Ala Ala Val Val Leu Ser Asn Gln Pro Gln Arg
                885                 890                 895

Asn Met Gln His Ala Met Asn Asn Leu Asn Leu Asn Leu His Gln His

900                 905                 910

Gly Gln Gln Thr Ala Ala Ala Ala Ala Ala Ala Ala Ser Ile Ala Ala
        915                 920                 925

Ala Leu Gln Gln His Ala Val Gln Ala Ser Asn Ala Ser Asn Asn Leu
    930                 935                 940

Asn Ile Ser His Asn Tyr Leu Gln Gln Gln His Val Gln Gln Gln Gln
945                 950                 955                 960

Gln Gln Gln Arg Gly Gln Gln Pro Gln Pro His Pro His Arg Asn His
                965                 970                 975

Asn Leu Asn Val Asp Gly Asn Gly Leu Asp Asn Thr Asn Asn Ile Ile
            980                 985                 990

Met Gln Ala Asn Met Asp Asp Leu Gln Tyr Lys Trp Trp Trp Cys Pro
        995                 1000                1005

Arg Ala Pro Arg Ser Ile Pro Ala Phe Thr Phe Ala Ser Trp Pro Ser
    1010                1015                1020

Ser Cys Ser Phe Ser Trp Ala Leu Asn Arg Pro Lys Gln Ala Phe Cys
025                 1030                1035                1040

Gly Phe Ile Thr Ile Phe Leu His Cys Ala Ile Leu Ser Gly Thr Ala
                1045                1050                1055

Trp Phe Cys Tyr Glu Ala Phe His Ser Tyr Ser Thr Leu Thr Ser Asp
            1060                1065                1070

Glu Leu Leu Leu Glu Val Asp Gln Thr Pro Lys Val Asn Cys Tyr Tyr
        1075                1080                1085

Leu Leu Ser Tyr Gly Leu Ser Leu Ser Val Val Ala Ile Ser Leu Val
    1090                1095                1100

Ile Asp Pro Ser Thr Tyr Thr Gln Asn Asp Tyr Cys Val Leu Met Glu
105                 1110                1115                1120

Ala Asn Ala Leu Phe Tyr Ala Thr Phe Val Ile Pro Val Leu Val Phe
                1125                1130                1135

Phe Val Ala Ala Ile Gly Tyr Thr Phe Leu Ser Trp Ile Ile Met Cys
            1140                1145                1150

Arg Lys Ser Arg Thr Gly Leu Lys Thr Lys Glu His Thr Arg Leu Ala

1155                1160                1165

Ser Val Arg Phe Asp Ile Arg Cys Ser Phe Val Phe Leu Leu Leu Leu
   1170                1175                1180

Ser Ala Val Trp Cys Ser Ala Tyr Phe Tyr Leu Arg Gly Ala Lys Met
185                 1190                1195                1200

Asp Asp Asp Thr Ala Asp Val Tyr Gly Tyr Cys Phe Ile Cys Phe Asn
                1205                1210                1215

Thr Leu Leu Gly Leu Tyr Ile Phe Val Phe His Cys Ile Gln Asn Glu
            1220                1225                1230

Lys Ile Arg Arg Glu Tyr Arg Lys Tyr Val Arg Gln His Ala Trp Leu
        1235                1240                1245

Pro Lys Cys Leu Arg Cys Ser Lys Thr Ser Ile Ser Ser Gly Ile Val
   1250                1255                1260

Thr Gly Asn Gly Pro Thr Ala Gly Thr Leu Cys Ser Val Ser Thr Ser
265                 1270                1275                1280

Lys Lys Pro Lys Leu Pro Leu Gly Val Ser Glu Glu Ala His Asp Asp
                1285                1290                1295

Pro Gln Gln Gln Gln Gln Thr Pro Val Pro Ile Thr Glu Asp Ala Ile
            1300                1305                1310

Met Gly Ala Thr Ser Asp Cys Glu Leu Asn Glu Ala Gln Gln Arg Arg
        1315                1320                1325

Thr Leu Lys Ser Gly Leu Met Thr Gly Thr Leu Gln Ala Pro Pro Gln
   1330                1335                1340

Thr Leu Gly Gly His Val Val Leu Glu Arg
345                 1350

<210> 5
<211> 20
<212> DNA
<213> Künstliche Sequenz

<220>
<223> Beschreibung der künstlichen Sequenz: Primer

<400> 5
tccatcgcca acgatatgtc                                                    20

<210> 6
<211> 20
<212> DNA
<213> Künstliche Sequenz

<220>
<223> Beschreibung der künstlichen Sequenz: Primer

<400> 6
cgctccctga tgatcgtatc                                                    20

## Beschreibung

Die Erfindung betrifft Polypeptide, welche die biologische Aktivität von Latrotoxin-Rezeptoren ausüben, sowie Nukleinsäuren, die für diese Polypeptide codieren und insbesondere deren Verwendung zum Auffinden von Wirkstoffen für den Pflanzenschutz.

Das Gift der Schwarzen Witwe (Latrodectus mactans tredecimguttatus) enthält eine Reihe hochpotenter Neurotoxine (Longenecker et al., 1970; Cull-Candy et al., 1973; Dulubova et al., 1996). Das bestuntersuchte Toxin aus dieser Reihe ist das alpha-Latrotoxin, welches zu einer massiven Ausschüttung von Neurotransmittern an Nervenendigungen sowohl in Vertebraten als auch in Invertebraten führt (Übersicht in Rosenthal und Meldolesi, 1989). Bei Insekten führt dies zu einer raschen Lähmung des Tiers (Cull-Candy et al., 1973).

Latrotoxin entfaltet seine Wirkung durch zwei prinzipiell unterschiedliche Mechanismen (Übersicht in Henkel und Sankaranarayanan, 1999), einem Calciumabhängigen und einem Calcium-unabhängigen Mechanismus. Der Calciumunabhängige Mechanismus bedarf eines Rezeptors in der Membran der Zielzelle. Dieser Rezeptor ist entweder Neurexin oder das Latrophilin (Übersicht in Henkel und Sankaranarayanan, 1999). Latrophilin gehört zur Klasse der G-Protein-gekoppelten Rezeptoren. Diese Rezeptoren binden üblicherweise an intrazelluläre Signalproteine, den G-Proteinen. Eine Aktivierung eines solchen Rezeptors durch Bindung eines Agonisten an der Außenseite der Zelle führt zu einer Aktivierung eines dieser intrazellulären G-Proteine, was eine Aktivierung spezifischer Signalkaskaden innerhalb der Zelle zur Folge hat. Im Falle des Latrophilins in Neuronen führt das daraufhin zu einer spontanen Ausschüttung von Neurotransmittern (Übersicht in Henkel und Sankaranarayanan, 1999).

Latrophilin existiert in Form verschiedener homologer Proteine, die durch alternatives Spleißen ("splicing") entstehen können. Unterschiedliche homologe Latrophiline können dabei in verschiedenen Organen und Geweben unterschiedlich exprimiert werden (Matsushita et al., 1999).

Um neuartige Insektizide anhand des Latrophilins zu entwickeln, können zwei Ansätze verfolgt werden. Zum einen kann nach Agonisten des Latrophilins gesucht werden, d.h. nach Verbindungen, die nach Bindung an Latrophilin intrazelluläre G-Proteine aktivieren. Zum anderen besteht die Möglichkeit, in Anwesenheit von Latrotoxin nach Inhibitoren der Aktivierung des Latrophilins zu suchen.

Der vorliegenden Erfindung liegt somit insbesondere die Aufgabe zugrunde, aus Insekten stammende Rezeptoren, an die alpha-Latrotoxin binden kann, und darauf aufbauende Testsysteme mit einem hohen Durchsatz an Testverbindungen (High Throughput Screening Assays; HTS-Assays) bereitzustellen.

Die Aufgabe wird gelöst durch die Bereitstellung von Polypeptiden, welche zumindest eine biologische Aktivität eines Latrotoxin-Rezeptors (Latrophilin) ausüben und eine Aminosäuresequenz umfassen, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit einer Sequenz gemäß SEQ ID NO: 2 oder SEQ ID NO: 4 über eine Länge von wenigstens 20, vorzugsweise wenigstens 25, besonders bevorzugt wenigstens 30 fortlaufenden Aminosäuren und ganz besonders bevorzugt über deren Gesamtlänge aufweisen.

Der Grad der Identität der Aminosäuresequenzen wird vorzugsweise bestimmt mit Hilfe des Programms GAP aus dem Programmpaket GCG, Version 9.1 unter Standardeinstellungen (Devereux et al., 1984).

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Amino-und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Die erfindungsgemäßen Polypeptide müssen nicht vollständige Rezeptoren darstellen, sondern können auch nur Fragmente davon sein, solange sie zumindest noch eine biologische Aktivität der vollständigen Rezeptoren aufweisen. Polypeptide, die im Vergleich zu Rezeptoren, die aus den erfindungsgemäßen Polypeptiden mit einer Aminosäuresequenz gemäß SEQ ID NO: 2 oder SEQ ID NO: 4 bestehen, eine um 50 % höhere oder verminderte Aktivität ausüben, werden noch als erfindungsgemäß betrachtet. Dabei müssen die erfindungsgemäßen Polypeptide nicht von Rezeptoren aus Drosophila melanogaster ableitbar sein. Als erfindungsgemäß werden auch Polypeptide betrachtet, die Rezeptoren beispielsweise der folgenden Invertebraten entsprechen oder Fragmenten davon, die noch die biologische Aktivität dieser Rezeptoren ausüben können: Insekten, Nematoden, Arthropoden, Mollusken.

Die erfindungsgemäßen Polypeptide können im Vergleich zu der entsprechenden Region natürlich vorkommender Rezeptoren Deletionen oder Aminosäuresubstitutionen aufweisen, solange sie zumindest noch eine biologische Aktivität der vollständigen Rezeptoren ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Der Ausdruck "biologische Aktivität eines Latrotoxin-Rezeptors", wie er hierin verwendet wird, bedeutet die Bindung von Latrotoxin an den Rezeptor.

Eine bevorzugte Ausführungsform der erfindungsgemäßen Polypeptide stellt ein Rezeptor von Drosophila melanogaster dar, welcher die Aminosäuresequenz gemäß SEQ ID NO: 2 oder SEQ ID NO: 4 besitzt.

Gegenstand der vorliegenden Erfindung sind auch Nukleinsäuren, die für die erfindungsgemäßen Polypeptide codieren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Eine bevorzugte Ausführungsform der erfindungsgemäßen Nukleinsäuren stellt eine cDNA dar, welche die Nukleinsäuresequenz gemäß SEQ ID NO: 1 oder SEQ ID NO: 3 besitzt.

Nukleinsäuren, welche unter stringenten Bedingungen an die Sequenz gemäß SEQ ID NO: 1 oder SEQ ID NO: 3 hybridisieren, sind ebenfalls von der vorliegenden Erfindung umfasst.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Insekten als Drosophila melanogaster isoliert werden, welche für Polypeptide mit der biologischen Aktivität von Rezeptoren codieren.

Bevorzugte Hybridisierungsbedingungen sind nachstehend angegeben:

Hybridisierungslösung: 6X SSC / 0 % Formamid, bevorzugte Hybridisierungslösung: 6X SSC / 25 % Formamid

Hybridisierungstemperatur: 34°C, bevorzugte Hybridisierungstemperatur: 42°C
1. Waschschritt: 2X SSC bei 40°C,
2. Waschschritt: 2X SSC bei 45°C; bevorzugter 2. Waschschritt: 0,6X SSC bei 55°C; besonders bevorzugter 2. Waschschritt: 0,3X SSC bei 65°C.

Weiterhin sind von der vorliegenden Erfindung Nukleinsäuren umfasst, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit der Sequenz gemäß SEQ ID NO: 1 oder SEQ ID NO: 3 über eine Länge von wenigstens 20, vorzugsweise wenigstens 25, besonders bevorzugt wenigstens 30 fortlaufenden Nukleotiden und ganz besonders bevorzugt über deren Gesamtlänge aufweisen.

Der Grad der Identität der Nukleinsäuresequenzen wird vorzugsweise bestimmt mit Hilfe des Programms GAP aus dem Programmpaket GCG, Version 9.1 unter Standardeinstellungen.

Gegenstand der vorliegenden Erfindung sind weiterhin DNA-Konstrukte, die eine erfindungsgemäße Nukleinsäure und einen heterologen Promotor umfassen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert. Der Ausdruck "Promotor", wie er hierin verwendet wird, bezieht sich allgemein auf Expressionskontrollsequenzen.

Die Auswahl von heterologen Promotoren ist davon abhängig, ob zur Expression pro- oder eukaryotische Zellen oder zellfreie Systeme verwendet werden. Beispiele für heterologe Promotoren sind der frühe oder späte Promotor des SV40, des Adenovirus oder des Cytomegalovirus, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des α-Mating-Faktors der Hefe.

Gegenstand der Erfindung sind weiterhin Vektoren, die eine erfindungsgemäße Nukleinsäure bzw. ein erfindungsgemäßes DNA-Konstrukt enthalten. Als Vektoren können alle in molekularbiologischen Laboratorien verwendete Plasmide, Phasmide, Cosmide, YACs oder künstliche Chromosomen verwendet werden.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, wie Bakterien der Gattungen Bacillus, Pseudomonas, Streptomyces, Streptococcus, Staphylococcus, vorzugsweise E. coli, als auch eukaryotische Zellen, wie Hefen, Säuger-, Amphibien-, Insekten- oder Pflanzenzellen. Bevorzugte eukaryotische Wirtszellen sind HEK-293-, Schneider S2-, Spodoptera Sf9-, Kc-, CHO-, COS1-, COS7-, HeLa-, C127-, 3T3- oder BHK-Zellen und insbesondere Xenopus-Oocyten.

Weiterhin sind Antikörper Gegenstand der Erfindung, die spezifisch an die vorstehend genannten Polypeptide bzw. Rezeptoren binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin läßt sich in an sich bekannter Weise eine immortalisierte Zellinie erhalten, die monoklonale Antikörper produziert. Die Antikörper können gegebenenfalls mit einem Nachweisreagenz markiert sein. Bevorzugte Beispiele für ein solches Nachweis-Reagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften besitzen. Daher erstreckt sich der Ausdruck "Antikörper", wie er hierin verwendet wird, auch auf Teile vollständiger Antikörper, wie Fa-, F(ab')₂- oder Fv-Fragmente, welche noch die Fähigkeit besitzen, an die Epitope der erfindungsgemäßen Polypeptide zu binden.

Die erfindungsgemäßen Nukleinsäuren können insbesondere zur Herstellung transgener Invertebraten verwendet werden. Diese können in Testsysteme eingesetzt werden, die auf einer vom Wildtyp abweichenden Expression der erfindungsgemäßen Polypeptide basieren. Ferner kann man auf der Grundlage der hierin offenbarten Informationen transgene Invertebraten herstellen, bei denen durch die Modifikation anderer Gene oder Promotoren eine Veränderung der Expression der erfindungsgemäßen Polypeptide eintritt.

Die Herstellung der transgenen Invertebraten erfolgt beispielsweise bei Drosophila melanogaster durch P-Element vermittelten Gentransfer (Hay et al., 1997) oder in Caenorhabditis elegans durch Transposon-vermittelten Gentransfer (z.B. durch Tc1; Plasterk, 1996).

Gegenstand der Erfindung sind somit auch transgene Invertebraten, die zumindest eine der erfindungsgemäßen Nukleinsäuren enthalten, vorzugsweise transgene Invertebraten der Arten Drosophila melanogaster oder Caenorhabditis elegans sowie deren transgene Nachkommen. Vorzugsweise enthalten die transgenen Invertebraten die erfindungsgemäßen Polypeptide in einer vom Wildtyp abweichenden Form.

Gegenstand der vorliegenden Erfindung sind weiterhin Verfahren zum Herstellen der erfindungsgemäßen Polypeptide. Zur Herstellung der Polypeptide, die von den erfindungsgemäßen Nukleinsäuren codiert werden, können Wirtszellen, die eine der erfindungsgemäßen Nukleinsäuren enthalten, unter geeigneten Bedingungen kultiviert werden. Dabei kann die zu exprimierende Nukleinsäure an die "Codon Usage" der Wirtszellen angepaßt werden. Die gewünschten Polypeptide können danach auf übliche Weise aus den Zellen oder dem Kulturmedium isoliert werden. Die Polypeptide können auch in *in vitro*-Systemen hergestellt werden.

Ein schnelles Verfahren zum Isolieren der erfindungsgemäßen Polypeptide, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkem zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Da die Rezeptoren Membranproteine darstellen, werden in den Reinigungsverfahren vorzugsweise Detergensextraktionen durchgeführt, beispielsweise unter Verwendung von Detergenzien, die die Sekundär- und Tertiärstrukturen der Polypeptide nicht oder nur wenig beeinflussen, wie nicht-ionische Detergenzien.

Die Reinigung der erfindungsgemäßen Polypeptide kann die Isolierung von Membranen ausgehend von Wirtszellen, die die erfindungsgemäßen Nukleinsäuren exprimieren, umfassen. Vorzugsweise exprimieren solche Zellen die erfindungsgemäßen Polypeptide in einer ausreichenden Kopienanzahl, so dass die Menge der Polypeptide in einer Membranfraktion mindestens 10-fach höher ist als diejenige, die in vergleichbaren Membranen von Zellen gefunden wird, die Rezeptoren natürlicherweise exprimieren; besonders bevorzugt ist Menge mindestens 100-fach, ganz besonders bevorzugt mindestens 1000-fach höher.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Ferner sind auch Verfahren zum Herstellen der erfindungsgemäßen Nukleinsäuren Gegenstand der vorliegenden Erfindung. Die erfindungsgemäßen Nukleinsäuren können auf die übliche Weise hergestellt werden. Beispielsweise können die Nukleinsäuremoleküle vollständig chemisch synthetisiert werden. Man kann auch nur kurze Stücke der erfindungsgemäßen Sequenzen chemisch synthetisieren und solche Oligonukleotide radioaktiv oder mit einem Fluoreszenzfarbstoff markieren. Die markierten Oligonukleotide können verwendet werden, um ausgehend von Insekten-mRNA hergestellte cDNA-Banken oder ausgehend von genomischer Insekten-DNA hergestellte genomische Banken zu durchsuchen. Klone, an die die markierten Oligonukleotide hybridisieren, werden zur Isolierung der betreffenden DNA ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Mit Hilfe der erfindungsgemäßen Nukleinsäuren bzw. Polypeptide können neue Wirkstoffe für den Pflanzenschutz bzw. pharmazeutische Wirkstoffe für die Behandlung von Mensch und Tier, wie chemische Verbindungen, welche als Modulatoren, insbesondere als Agonisten oder Antagonisten, die Eigenschaften der erfindungsgemäßen Rezeptoren verändern, identifiziert werden. Dazu wird ein rekombinantes DNA-Molekül, das zumindest eine erfindungsgemäße Nukleinsäure umfasst, in eine geeignete Wirtszelle eingebracht. Die Wirtzelle wird in Gegenwart einer Verbindung oder einer Probe, welche eine Vielzahl von Verbindungen umfasst, unter Bedingungen kultiviert, die die Expression der erfindungsgemäßen Rezeptoren erlauben. Eine Veränderung der Rezeptoreigenschaften kann beispielsweise wie nachstehend in Beispiel 2 beschrieben detektiert werden. Auf diese Weise ist es möglich, beispielsweise insektizide Substanzen aufzufinden.

Rezeptoren verändern vorzugsweise nach Aktivierung die Konzentration von intrazellulärem cAMP über eine Interaktion mit G-Proteinen. Veränderungen der Rezeptoreigenschaften durch chemische Verbindungen können deshalb nach heterologer Expression zum Beispiel durch Messung der intrazellulären cAMP-Konzentrationen direkt über ELISA-Assaysysteme (Biomol, Hamburg, Deutschland) oder RIA-Assaysysteme (NEN, Schwalbach, Deutschland) im HTS-Format gemessen werden. Eine indirekte Messung der cAMP-Konzentration ist mit Hilfe von Reportergenen (z.B. Luziferase) möglich, deren Expression von der cAMP-Konzentration abhängig ist (Stratowa et al., 1995). Die Koexpression von Rezeptoren mit besonderen G-Proteinen, z.B. Gα15, Gα16 oder auch chimären G-Proteinen, in heterologen Systemen und die Messung des Anstiegs von Calcium, z.B. mit Fluoreszenzfarbstoffen oder Äquorin, ist eine alternative Screeningmöglichkeit (Stables et al., 1997; Conklin et al., 1993).

Weiterhin kann die Bindung von GTP an das aktivierte G-Protein als read-out-System für die Prüfung von Substanzen herangezogen werden. Auch Bindungsexperimente mit markiertem Peptiden können zum Screening eingesetzt werden.

Der Ausdruck "Agonist", wie er hierin verwendet wird, bezieht sich auf ein Molekül, das den Rezeptor aktiviert.

Der Ausdruck "Antagonist", wie er hierin verwendet wird, bezieht sich auf ein Molekül, das einen Agonisten von seiner Bindungsstelle verdrängt.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können Mimetika von natürlichen Substraten und Liganden darstellen.

Vorzugsweise handelt es sich bei den Modulatoren um kleine organisch-chemische Verbindungen.

Die Bindung der Modulatoren an die erfindungsgemäßen Polypeptide kann die zellulären Vorgänge auf eine Weise verändern, die zum Absterben der damit behandelten Insekten führt.

Daher erstreckt sich die vorliegende Erfindung auch auf die Verwendung von Modulatoren der erfindungsgemäßen Polypeptide als Insektizide oder Arzneimittel.

Die erfindungsgemäßen Nukleinsäuren bzw. Polypeptide ermöglichen auch das Auffinden von Verbindungen, die an die erfindungsgemäßen Rezeptoren binden. Diese können ebenfalls als Insektizide auf Pflanzen bzw. pharmazeutische Wirkstoffe für die Behandlung von Mensch und Tier angewandt werden. Beispielsweise werden Wirtszellen, die die erfindungsgemäßen Nukleinsäuren enthalten und die entsprechenden Rezeptoren bzw. Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Rezeptoren oder den einzelnen Polypeptiden erlauben.

Unter Verwendung von Wirtszellen oder transgenen Invertebraten, die die erfindungsgemäßen Nukleinsäuren enthalten, ist es auch möglich, Substanzen aufzufinden, welche die Expression der Rezeptoren verändern.

Die vorstehend beschriebenen erfindungsgemäßen Nukleinsäuren, Vektoren und regulatorischen Regionen können außerdem zum Auffinden von Genen verwendet werden, die für Polypeptide codieren, welche am Aufbau von funktionell ähnlichen Rezeptoren in Insekten beteiligt sind. Unter funktionell ähnlichen Rezeptoren werden gemäß der vorliegenden Erfindung Rezeptoren verstanden, die Polypeptide umfassen, welche sich zwar hinsichtlich der Aminosäuresequenz von den hierin beschriebenen Polypeptiden unterscheiden, aber im wesentlichen dieselben Funktionen haben.

**Erläuterungen zum Sequenzprotokoll und zu der Abbildung:**

SEQ ID NO: 1 und SEQ ID NO: 3 zeigen die Nukleotid- und Aminosäuresequenzen der isolierten Rezeptor-cDNA. SEQ ID NO: 2 und SEQ ID NO: 4 zeigen ferner die Aminosäuresequenzen der von den Rezeptor-cDNA-Sequenzen abgeleiteten Proteine.

SEQ ID NO: 5 zeigt die Sequenz des Primers 1s.

SEQ ID NO: 6 zeigt die Sequenz des Primers 1a.

### Beispiele:

### Beispiel 1

Isolierung des beschriebenen Polynukleotids

Die Manipulation von Polynukleotiden erfolgte nach Standardmethoden der rekombinanten DNA Technologie (Sambrook et al., 1989). Die bioinformatische Bearbeitung von Nukleotid- und Proteinsequenzen erfolgten mit dem Programmpaket GCG Version 9.1 (GCG Genetics Computer Group, Inc., Madison Wisconsin, USA).

Isolierung von poly A enthaltender RNA aus Drosophila-Gewebe und Konstruktion der cDNA-Bibliotheken

Die RNA für die cDNA-Bibliothek I wurde aus gesamten Drosophila melanogaster Embryonen und Larven (RNAzo1, Life Technologies, Karlsruhe, Deutschland, nach Angaben des Herstellers) isoliert. Aus dieser RNA wurden nun die poly A enthaltenden RNAs durch Reinigung über Dyna Beads 280 (Dynal, Hamburg, Deutschland) isoliert. 5 µg dieser poly A enthaltenden RNAs wurden anschließend in die Konstruktion der cDNA-Bibliothek mit dem λ-ZAP-CMV Vektor eingesetzt (cDNA Synthesis Kit, ZAP-cDNA Synthesis Kit und ZAP-cDNA Gigapack III Gold Cloning Kit, alle Stratagene-Europe, Amsterdam, Niederlande).

### Erzeugung von Plasmid Pools

Die cDNA-Bibliothek in Lambda-pCMV wurde nach Angaben des Herstellers einer Massen in vivo Exzision unterzogen, um eine Phagemid-Bibliothek zu erzeugen. Anschließend wurden 10 x 96 Minipräparations-Kulturen angeimpft, die rechnerisch 1000 Klone pro Präparation enthielten. Die DNA wurde dann mit dem Qiawell Ultra DNA Präparationssystem von Qiagen (Hilden, Deutschland) aufgereinigt und in 96-Loch-Mikrotiterplatten abgelegt. Dadurch wurde die Bibliothek in Form von 960 Pools ä 1000 cDNA Klonen abgebildet.

### PCR mit Bibliothek-Pools

Von jeder Mikrotiterplatte wurde ein Meta-Pool angelegt, der die gesamte Platte repräsentierte. Je 0,5 µl dieses Metapools wurde in eine PCR mit den folgenden Oligodesoxynukleotid-Primern eingesetzt:

Primer 1s :TCCATCGCCAACGATATGTC (SEQ ID NO: 5)

Primer 1a :CGCTCCCTGATGATCGTATC (SEQ ID NO: 6)

Die PCR-Parameter waren wie folgt: 94°C, 1 min; 35 mal (94°C, 30 s; 55°C, 30 s; 72°C, 45 s). Die PCRs liefen auf einem Biometra Uno II (Biometra, Göttingen, Deutschland).

In der PCR positive Bibliothek-Pools wurden in X1-1Blue (Stratagene, Amsterdam, Niederlande) transformiert und einem Kolonie-Lift unterzogen (Sambrook et al., 1989). Als Sonde für die Hybridisierung diente ein mittels Psoralen-Biotin (BrightStar, Psoralen-Biotin Kit, Ambion, Austin, Texas, USA) markiertes PCR-Produkt aus der Reaktion mit dem jeweiligen Primerpaar (Hybridisierung und Detektion mittels BrightStar, Psoralen-Biotin Kit, Ambion, Austin, Texas, USA). Positive Kolonien wurden gepickt, vermehrt und die DNA per Plasmid-Präparation (Qiagen, Hilden, Deutschland) isoliert.

Die isolierten Genbankplasmide wurden zur Identifikation mittels T3-und T7-Primer ansequenziert (ABI Prism Dye Terminator Cycle Sequencing Kit, ABI, mit ABI Prism 310 Genetic Analyzer, ABI-Deutschland, Weiterstadt, Deutschland). Die Bestimmung der vollständigen Polynukleotidsequenzen des DB3 erfolgte durch Primer Walking mittels Cycle Sequencing ABI Prism Dye Terminator Cycle Sequencing Kit, ABI, mit ABI Prism 310 Genetic Analyzer (ABI-Deutschland, Weiterstadt, Deutschland).

### Beispiel 2

Die Zuordnung der Sequenzen gemäß SEQ ID NO: 2 und SEQ ID NO: 4 erfolgte mittels Blast-Analyse (Blastp; Altschul et al., 1997). Gezeigt ist der beste Treffer aus der Blast-Analyse (Nicht-redundante Protein Datenbank: Genbank CDS translations + PDB + SwissProt + PIR Datenbank vom 4. März 2000). Der Parameter E-value ist ein Maß für die Nichtzufälligkeit der Zuordnung. Die Sequenz konnte mit hinreichender Sicherheit als Latrotoxin-Rezeptor identifiziert werden.

### Sequenzvergleich und Zuordnung der Sequenz

| **Seq ID** | **E-value** | **Accession No. / Accession aus Swissprot Datenbank (vom 4. März 2000)** |
|---|---|---|
| 2 | 6e-65 | AF111098 / Latrophilin-1 (Bos taurus) |
| 4 | 4e-45 | AF111098 / Latrophilin-1 (Bos taurus) |

### Beispiel 3

### Heterologe Expression

Eine funktionelle Expression des erfindungsgemäßen Rezeptors aus Insekten kann in Xenopus-Oocyten erfolgen. Dazu werden G-Protein aktivierbare Kalium-Kanäle (GIRK1 und GIRK4) koexprimiert, um die Aktivierung der Rezeptoren zu messen (White et al., 1998). Die verwendungsgemäße Nukleinsäure wird, da sie sich bereits in einem Expressionsvektor mit CMV-Promoter befindet, direkt in die Expressionsversuche eingesetzt.

### Oocyten-Messungen

### 1. Präparation der Oocyten

Die Oocyten stammen von einem adulten weiblichen *Xenopus laevis*-Frosch (Firma Horst Kähler, Hamburg, Deutschland). Die Frösche werden in großen Tanks mit zirkulierendem Wasser bei einer Wassertemperatur von 20 - 24°C gehalten. Teile des Frosch-Ovars werden unter vollständiger Anaesthesie durch einen kleinen Schnitt im Abdomen (ca. 1cm) entnommen. Anschließend wird das Ovar unter ständigem Schütteln für ca. 140min in 25ml Kollagenase (Typ I, C-0130, SIGMA-ALDRICH CHEMIE GmbH, Deisenhofen, Deutschland; 355U/ml, angesetzt in Barth's Lösung ohne Calcium in mM: NaCl 88, KCl 1, MgSO4 0.82, NaHCO3 2.4, Tris/HCl 5, pH7.4) behandelt. Die Oocyten werden dann mit Barth's Lösung ohne Calcium gewaschen. Nur Oocyten im Reifestadium V (Dumont, 1972) werden für die weitere Behandlung ausgewählt und in Mikrotiterplatten (Nunc Micro Well™ Platten, Kat. Nr. 245128 + 263339 (Deckel), Nunc GmbH & Co. KG, Wiesbaden, Deutschland), gefüllt mit Barth's-Lösung (in mM: NaCl 88, KCl 1, MgSO₄ 0.82, Ca(NO₃)₂ 0.33, CaCl₂ 0.41, NaHCO3 2.4, Tris/HCl 5, pH7.4) sowie Gentamicin (Gentamicin Sulfate, G-3632, SIGMA-ALDRICH CHEMIE GmbH, Deisenhofen, Deutschland; 100U/ml), überführt. Die Oocyten werden dann bei 19.2°C in einem Kühl-Brutschrank (Typ KB 53, WTB Binder Labortechnik GmbH, Tuttlingen, Deutschland) aufbewahrt.

### 2. Injektion der Oocyten

Injektionselektroden, mit einem Durchmesser von 10 - 15µm, werden mit einem Pipetten-Puller (Typ L/M-3P-A, List-electronic, Darmstadt-Eberstadt, Deutschland) hergestellt. Vor der Injektion werden Aliquots mit der Rezeptor-DNA bzw. GIRK1/4-DNA aufgetaut und mit Wasser auf eine Endkonzentration von 10 ng/µl verdünnt. Die DNA-Proben werden mit 3200 g für 120s zentrifugiert (Typ Biofuge 13, Heraeus Instruments GmbH, Hanau, Deutschland). Anschließend wird ein ausgezogener PE-Schlauch als Transferschlauch benutzt, um die Pipetten von hinten zu befüllen. Die Injektionselektroden werden an einer X,Y,Z-Verfahreinheit (Bearbeitungszentrum EP1090, isel-automation, Eiterfeld, Deutschland) befestigt. Mit Hilfe eines Macintosh Computer wird die Oocyten in den Vertiefungen der Mikrotiterplatten angefahren und durch kurze Druckapplikation (0.5-3.0bar, 3-6s) ca. 50nl der DNA-Lösung in die Oocyte injiziert.

### 3. Elektrophysiologische Messungen

Für die elektrophysiologischen Messungen wird eine Zwei-Elektroden-Spannungsklemme mit einem TURBO TEC-10CD (npi electronic GmbH, Tamm, Deutschland) Verstärker durchgeführt. Die hierfür notwendigen Mikropipetten werden aus Aluminiumsilikatglas (Kapillarrohr, Art.-Nr. 14 630 29, 1=100mm, Øa=1,60mm, Øi=1,22mm, Hilgenberg GmbH, Malsfeld, Deutschland) in zwei Zügen gezogen (Hamill et al., 1981). Strom- und Spannungselektroden haben einen Durchmesser von 1 - 3µm und werden mit 1.5M KCl und 1.5M Kaliumacetat gefüllt. Die Pipetten haben einen kapazitiven Widerstand von 0.2 - 0.5 MW. Für die elektrophysiologischen Messungen werden die Oocyten in eine kleine Kammer überführt, die kontinuierlich mit normaler Rimland-Lösung (in mM: KCl 90, MgCl₂ 3, HEPES 5, pH 7.2) gespült wird. Für eine Substanzapplikation wird die Perfusionslösung durch eine Substanzlösung mit gleicher Zusammensetzung und zusätzlich der gewünschten Substanzkonzentration ausgetauscht. Bei einem Klemmpotential von -60mV wird die erfolgreiche Expression der Rezeptor-DNA nach einer Woche überprüft. Nicht reagierende Oocyten werden verworfen. Alle weiteren werden für die Substanztestung verwendet. Die Daten werden mittels eines YT-Schreibers (YT- Schreiber, Model BD 111, Kipp & Zonen Delft BV, AM Delft, Niederlande) dokumentiert. Wenn Testsubstanzen in Konzentrationsreihen untersucht werden, werden diese Messungen an mindestens zwei verschiedenen Oocyten und an mindestens fünf verschiedenen Konzentrationen durchgeführt. Die Substanzen werden direkt und ohne Vorinkubation in Gegenwart von Glutamat (Gamma-Amino-N-butyric acid, A2129, SIGMA-ALDRICH CHEMIE GmbH, Deisenhofen, Deutschland) auf ihren Antagonismus geprüft. Die einzelnen Werte werden in Origin (Auswertesoftware Microcal Origin, Microcal Software, Inc., Northampton, MA 01060-4410 USA (Additive GmbH, Friedrichsdorf/Ts, Deutschland) eingegeben. Mittelwerte, Standardabweichung, IC₅₀-Werte und IC₅₀-Kurven werden mit Origin berechnet. Diese Messungen werden mindestens zweimal durchgeführt.

### Literatur:

Altschul et al. (1997), Gapped BLAST and PSI-BLAST: a new generation of protein database search programs, Nucleic Acids Res. 25, 3389-3402.

Conklin et al. (1993), Substitution of three amino acids switches receptor specificity of Gq alpha to that of Gi alpha, Nature 363, 274-276

Cull-Candy et al. (1973), Nature 241, 353-354

Devereux et al. (1984), Nucleic Acids Research 12, 387

Dulubova et al. (1996), Cloning and structure of delta-lateroinsectotoxin, a novel insect-specific member of the latrotoxin family, J. Biol. Chem. 271 (13), 7535-7543

Dumont, J.N. (1972), Oogenesis in *Xenopus laevis* (Daudin). 1. Stages of oocyte development in laboratory maintained animals, J. Morphol. 136, 153-180 Hamill, O.P., Marty, A., Neher, E., Sakmann, B. Sigworth, F.J. (1981), Improved patch-clamp techniques for high-resolution current recording from cells and cell-free membrane patches, Pfügers Arch. 391, 85-100

Hay et al. (1997), P element insertion-dependent gene activation in the Drosophila eye, Proceedings of The National Academy of Sciences of The United States of America 94 (10), 5195-5200

Henkel und Sankaranarayanan (1999), Mechanisms of alpha-latrotoxin action, Cell Tissue Res. 296, 229-233

Longenecker et al. (1970), Nature 225, 701-703

Matsushita et al. (1999), The latrophilin family: multiply spliced G protein-coupled receptors with differential tissue distribution, FEBS Letters 443, 348-342

Plasterk (1996), The Tc1/mariner transposon family, Transposable Elements/Current Topics in Microbiology and Immunology 204, 125-143

Rosenthal und Meldolesi (1989), alpha-Latrotoxin and related toxins, Parmacol. Ther. 42, 115-134

Sambrook et al. (1989), Molecular Cloning, A Laboratory Manual, 2nd ed. Cold Spring Harbour Press

Stables et al. (1997), A Bioluminescent Assay for Agonist Activity at Potentially Any G-protein coupled Receptor, Analytical Biochemistry 252, 115-126

Stratowa C. et al. (1995), Use of a luciferase reporter system for characterizing G-protein-linked receptors, Current Opinion in Biotechnology 6, 574-581

White J. H. et al. (1998), Heterodimerization is required for the formation of a functional GABA(B) receptor, Nature 396, 679-682

## Patentansprüche

1. Polypeptid, welches die biologische Aktivität eines Latrotoxin-Rezeptors ausübt und eine Aminosäuresequenz umfasst, die eine zumindest 70 %ige Identität mit einer Sequenz gemäß SEQ ID NO:2 oder SEQ ID NO:4 aufweist.

2. Polypeptid gemäß Anspruch 1, **dadurch gekennzeichnet, dass** die Aminosäuresequenz einer Sequenz gemäß SEQ ID NO:2 oder SEQ ID NO:4 entspricht.

3. Nukleinsäure umfassend eine Nukleotidsequenz, die für ein Polypeptid gemäß Anspruch 1 oder 2 codiert.

4. Nukleinsäure gemäß Anspruch 3, **dadurch gekennzeichnet, dass** es sich um einzelsträngige oder doppelsträngige DNA oder RNA handelt.

5. Nukleinsäure gemäß Anspruch 4, **dadurch gekennzeichnet, dass** es sich um Fragmente genomischer DNA oder cDNA handelt.

6. Nukleinsäure gemäß Anspruch 3, **dadurch gekennzeichnet, dass** die Nukleotidsequenz einer Sequenz gemäß SEQ ID NO:1 oder SEQ ID NO:3 entspricht.

7. Nukleinsäure gemäß Anspruch 3, **dadurch gekennzeichnet, dass** sie unter stringenten Bedingungen an die Sequenzen gemäß SEQ ID NO:1 oder SEQ ID NO:3 hybridisiert.

8. DNA-Konstrukt umfassend eine Nukleinsäure gemäß einem der Ansprüche 3 bis 7 und einen heterologen Promotor.

9. Vektor umfassend eine Nukleinsäure gemäß einem der Ansprüche 3 bis 7 oder ein DNA-Konstrukt gemäß Anspruch 8.

10. Vektor gemäß nach Anspruch 9, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

11. Wirtszelle enthaltend eine Nukleinsäure gemäß einem der Ansprüche 3 bis 7, ein DNA-Konstrukt gemäß Anspruch 8 oder einen Vektor gemäß Anspruch 9 oder 10.

12. Wirtszelle gemäß Anspruch 11, **dadurch gekennzeichnet, dass** es sich um eine prokaryotische Zelle, insbesondere um E. coli, handelt.

13. Wirtszelle gemäß Anspruch 11, **dadurch gekennzeichnet, dass** es sich um eine eukaryotische Zelle, insbesondere um eine Säuger- oder Insektenzelle, handelt.

14. Antikörper, welcher spezifisch an ein Polypeptid gemäß Anspruch 1 oder 2 bindet.

15. Transgener Invertebrat enthaltend eine Nukleinsäure gemäß einem der Ansprüche 3 bis 7.

16. Transgener Invertebrat nach Anspruch 15, **dadurch gekennzeichnet, dass** es sich um Drosophila melanogaster oder Caenorhabditis elegans handelt.

17. Transgene Nachkommen eines Invertebraten gemäß Anspruch 15 oder 16.

18. Verfahren zum Herstellen eines Polypeptids gemäß Anspruch 1 oder 2, umfassend
(a) das Kultivieren einer Wirtszelle gemäß einem der Ansprüche 11 bis 13 unter Bedingungen, die die Expression der Nukleinsäure gemäß einem der Ansprüch 3 bis 7 gewährleisten, oder
(b) das Exprimieren einer Nukleinsäure gemäß einem der Ansprüche 3 bis 7 in einem *in vitro*-System, und
(c) die Gewinnung des Polypeptids aus der Zelle, dem Kulturmedium oder dem *in vitro*-System.

19. Verfahren zum Herstellen einer Nukleinsäure gemäß einem der Ansprüche 3 bis 7, umfassend die folgenden Schritte:
(a) Vollständige chemische Synthese auf an sich bekannte Weise, oder
(b) chemische Synthese von Oligonukleotiden, Markieren der Oligonukleotide, Hybridisieren der Oligonukleotide an DNA einer genomischen oder cDNA-Bank, die ausgehend von genomischer DNA bzw. mRNA aus Insektenzellen hergestellt wurde, Selektieren von positiven Klonen und Isolieren der hybridisierenden DNA aus positiven Klonen, oder
(c) chemische Synthese von Oligonukleotiden und Amplifizierung der Ziel-DNA mittels PCR.

20. Verfahren zum Herstellen eines transgenen Invertebraten gemäß Anspruch 15 oder 16, umfassend das Einbringen einer Nukleinsäure gemäß einem der Ansprüche 3 bis 7 oder eines Vektors gemäß Anspruch 9 oder 10.

21. Verfahren zum Auffinden neuer Wirkstoffe für den Pflanzenschutz, insbesondere von Verbindungen, welche die Eigenschaften von Polypeptiden gemäß Anspruch 1 oder 2 verändern, umfassend die folgenden Schritte:
(a) Bereitstellen einer Wirtszelle gemäß einem der Ansprüche 11 bis 13,
(b) Kultivieren der Wirtszelle in der Gegenwart einer chemischen Verbindung oder eines Gemischs von chemischen Verbindungen, und
(c) Detektieren veränderter Eigenschaften.

22. Verfahren zum Auffinden einer chemischen Verbindung, die an ein Polypeptid gemäß Anspruch 1 oder 2 bindet, umfassend die folgenden Schritte:
(a) Inkontaktbringen eines Polypeptids gemäß Anspruch 1 oder 2, oder einer Wirtszelle gemäß einem der Ansprüche 11 bis 13 mit einer chemischen Verbindung oder einem Gemisch von chemischen Verbindungen unter Bedingungen, die die Interaktion einer chemischen Verbindung mit dem Polypeptid erlauben, und
(b) Bestimmen der chemischen Verbindung, die spezifisch an das Polypeptid bindet.

23. Verfahren zum Auffinden einer chemischen Verbindung, die die Expression eines Polypeptids gemäß Anspruch 1 oder 2 verändert, umfassend die folgenden Schritte:
(a) Inkontaktbringen einer Wirtszelle gemäß einem der Ansprüche 11 bis 13 oder eines transgenen Invertebraten gemäß Anspruch 15 oder 16 mit einer chemischen Verbindung oder einem Gemisch von chemischen Verbindungen,
(b) Bestimmen der Konzentration des Polypeptids gemäß Anspruch 1 oder 2, und
(c) Bestimmen der chemischen Verbindung, die die Expression des Polypeptids spezifisch beeinflusst.

24. Verwendung eines Polypeptids gemäß Anspruch 1 oder 2, einer Nukleinsäure gemäß einem der Ansprüche 3 bis 7, eines Vektors gemäß Anspruch 9 oder 10, einer Wirtszelle gemäß einem der Ansprüche 11 bis 13, eines Antikörpers gemäß Anspruch 14 oder eines transgenen Invertebraten gemäß Anspruch 15 oder 16 zum Auffinden neuer Wirkstoffe für den Pflanzenschutz oder zum Auffinden von Genen, die für Polypeptide codieren, welche am Aufbau von funktionell ähnlichen Peptid-Rezeptoren in Insekten beteiligt sind.

25. Verwendung eines Modulators eines Polypeptids gemäß Anspruch 1 oder 2 als Insektizid.
